# EUROPEAN PATENT SPECIFICATION

(11) **EP 1 379 308 B1**
(45) Date of publication and mention of the grant of the patent: **17.12.2008**
(21) Application number: 02723789.0
(22) Date of filing: 08.04.2002
(51) Int. Cl.: A61M 25/00

(54) **CATHETER SLIT VALVES**
SCHLITZVENTILE FÜR KATHETER
VALVES FENDUES DE CATHETER

(30) Priority: 19.04.2001 US 838618
(43) Date of publication of application: 14.01.2004
(73) Proprietor: Navilyst Medical, Inc., Marlborough, MA 01752-1234 (US)
(72) Inventor: HAARALA, Brett, T., Framingham, MA 01701 (US); DRISCOLL, Arthur, Somerville, MA 02143 (US)
(74) Representative: Lawrence, John
(86) International application number: PCT/US2002/010890
(87) International publication number: WO 2002/085439

(56) References cited:
- EP-A- 0 328 332
- EP-A- 0 613 696
- US-A- 4 685 905
- US-A- 5 085 635
- US-A- 5 147 318
- US-A- 5 224 938
- US-A- 5 928 203
- US-A- 6 052 612

## Description

### Technical Field

The invention generally relates to.medical infusion and aspiration of fluids through a catheter, and in particular to improved catheter slit valves for medical fluid infusion and aspiration.

### Background Information

Infusion of fluid into the body or aspiration of fluid from the body is often performed with a catheter inserted beneath the skin. The catheter has a lumen through which fluid can flow. In some designs, the lumen is closed at the insertion end of the catheter and fluid communication between the body environment outside the catheter and the lumen is controlled by a slit through the catheter wall, which acts as a valve. The closed end of the catheter prevents the free migration of fluids into the lumen and retains the fluid content of the lumen, thereby reducing development of occlusions or other complications. In some designs, the catheter has a hub on the end outside the body, which can be connected to a syringe for infusion and/or aspiration of fluids to and from the body. The fluids are infused or aspirated by increasing or decreasing the pressure inside the lumen.

For infusion, the fluid pressure inside the lumen is increased to force the catheter body adjacent the slit to flex outwardly, separating the opposing faces of the slit, and forming an aperture through which fluid may pass to the body environment. For aspiration, the pressure inside the lumen is decreased to force the catheter body adjacent the slit to collapse inwardly, forming an aperture through which fluid may flow into the lumen. At neutral pressures, the catheter body assumes an unflexed condition in which the faces of the slit are opposed, which forms a seal to prevent infusion or aspiration.

A valve can be made to permit infusion only, aspiration only, or both infusion and aspiration. A valve that operates for infusion only can be formed by making the slit in a convex catheter wall portion, since the convex shape facilitates flexing outwardly while resisting flexing inwardly. A valve that operates for aspiration only can be formed by making the slit in a concave wall portion which facilitates flexing inwardly while resisting flexing outwardly. A flat wall portion facilitates flexing in either direction and can be used to form a two-direction valve.

A two-direction valve may also be formed by chemical weakening of the catheter wall adjacent the slit, which facilitates flexing in both directions so that the valve works smoothly during infusion and aspiration. The lumen may also be shaped with a linear side that terminates to form regions of reduced catheter wall thickness. The regions act as hinges at which inward and outward flexing is enhanced and the area between the regions may have a greater wall thickness to facilitate sealing. The catheter may also have multiple valves and multiple lumens.

The foregoing slit valves suffer from a number of limitations. For example, the narrow valve opening makes it difficult to aspirate blood out of a vein and restricts infusion, e.g., fluid flow into the vein. Also, the foregoing valves can require high pressure differentials to operate and do not always actuate reliably.
A device according to the preamble of claim 1 is known from US-A-5 224 938.

### SUMMARY OF THE INVENTION

The improved catheter slit valves of the invention use nonradial slits to address the problems found with conventional radial slit valves, e.g., difficultly in aspiration. The nonradial slit valves are more efficient than radial slit valves, because the geometry of the nonradial slit allows the catheter material to move apart with less interference or friction from the opposing wall. For example, the nonradial slit allows for easier aspiration by reducing the pressure required to open the valve, because less displacement of the wall is required to move the adjacent wall segments apart.

In addition, other variations of the improved catheter slit valves allow for easier infusion and/or aspiration and greater flow. For example, dual slit variations create a more flexible portion of the catheter wall which must move in order to aspirate and provide up to twice the open area of a single slit, for infusion and/or aspiration. Nonlinear slits make for more flexible wall portions that can be more easily moved under similar internal pressures, as well as providing larger apertures for fluid flow when activated. Also, nonradial slits can be used with catheters having protuberances disposed thereon. The slit is disposed adjacent or through the protuberance, and the protuberance acts to either stiffen one side of the valve or alter the effect of the pressure vectors on the opening and closing of the valve. For a slit disposed adjacent a protuberance, the protuberance will stiffen one side of the slit while the opposite side will yield reliably giving a more defined aperture.

According to the present invention there is provided a medical device comprising:
an elongate catheter including an external surface and at least one internal surface defining an internal lumen that extends longitudinally along at least a portion of the elongate catheter; and
a nonradial slit extending between end points separated along a portion of a length of the catheter, the nonradial slit extending through a wall of the catheter from the external surface to the at least one internal surface and into communication with the internal lumen, the nonradial slit being oriented so that, if a surface defined by the slit were extended through the catheter, the surface would not intersect an axis of the catheter.

In one aspect, the invention relates to a medical device including an elongate catheter including an external surface and one or more internal surfaces defining an internal lumen that extends longitudinally along at least a portion of the elongate catheter and a nonradial slit extending from the external surface to the one or more internal surfaces and into communication with the internal lumen.

In some embodiments the slit is generally longitudinally disposed on the elongate catheter and is either linear or nonlinear. A nonlinear slit may include at least one angle or be curved. The curved slit may have a radius between about 0.25 cm (0.10 inch) and about 1,27 cm (0.50 inch). In additional embodiments, the device includes a second nonradial slit extending through the external surface to the at least one internal surface and into communication with the internal lumen. Also, the lumen may be eccentric with respect to the external surface of the elongate catheter and the elongate catheter may include a second lumen. In the embodiment where the lumen is eccentric, the slit is preferably disposed in an area of the catheter having a thickened wall portion. Further, the elongate catheter may include a laminate disposed on the external surface extending from the slit up to about 225 degrees from the slit. Preferably, the laminate is disposed from about 45 degrees to about 225 degrees from the slit.

In another aspect, the invention relates to a medical device including an elongate catheter including an external surface and one or more internal surfaces defining an internal lumen that extends longitudinally along at least a portion of the elongate catheter, a protuberance disposed on the elongate catheter, and a nonradial slit extending from the external surface, through the protuberance to the one or more internal surfaces, and into communication with the internal lumen.

In some embodiments the protuberance and slit are generally longitudinally disposed on the catheter body. The protuberance is disposed on the external surface of the elongate catheter or the at least one internal surface of the elongate catheter or both. In some other embodiments, the device includes a second protuberance disposed on the elongate catheter and a second nonradial slit extending from the external surface, through the second protuberance to the at least one internal surface, and into communication with the internal lumen. Also, the first and second protuberances may be disposed opposite each other.

In yet another aspect, the invention relates to a medical device including an elongate catheter including an external surface and one or more internal surfaces defining an internal lumen that extends longitudinally along at least a portion of the elongate catheter, a protuberance disposed on the elongate catheter, and a slit disposed adjacent the protuberance. The slit extends from the external surface to the one or more internal surfaces, and into communication with the internal lumen.

In some embodiments the protuberance and slit are generally longitudinally disposed on the catheter body, and the protuberance is disposed on the external surface of the elongate catheter or the at least one internal surface of the elongate catheter or both. In some other embodiments, the device includes a second protuberance disposed on the elongate catheter and a second slit disposed adjacent the second protuberance and extending from the external surface to the at least one internal surface and into communication with the internal lumen. Also, the first and second protuberances may be disposed opposite each other and the slits may be nonradial.

In still another aspect, the invention relates to a medical device including an elongate catheter including an external surface and at least two internal surfaces defining two internal lumens that extend longitudinally along at least a portion of the elongate catheter, a first protuberance disposed on the elongate catheter and a first slit disposed adjacent the first protuberance and extending from the external surface to one of the internal surfaces, and into communication with the first internal lumen, and a second protuberance disposed on the elongate catheter and a second slit disposed adjacent the second protuberance and extending from the external surface to another of the internal surfaces, and into communication with the second internal lumen.

In some embodiments the protuberances and slits are generally longitudinally disposed on the catheter body, and the protuberances may be disposed on the external surface of the elongate catheter or the at least one internal surface of the elongate catheter or both. Also, the first and second protuberances may be disposed opposite each other and the slits may be nonradial.

In yet another aspect, the invention relates to a medical device including an elongate catheter defining a first lumen that extends longitudinally along at least a portion of the elongate catheter, a cap disposed at a distal end of the elongate catheter and including an external surface and at least one internal surface defining a second lumen, and a nonradial slit that extends from the external surface of the cap to the at least one internal surface of the cap and into communication with the first and second lumens.

Still a further aspect of the invention relates to a medical device including an elongate catheter including an external surface and at least two internal surfaces defining two internal lumens that extend longitudinally along at least a portion of the elongate catheter, a first nonradial slit extending from the external surface to one of the internal surfaces and into communication with the first internal lumen, and a second nonradial slit extending from the external surface to another of the internal surfaces and into communication with the second internal lumen.

In various embodiments the nonradial slit forms an included angle with a radial line between about 1 degree and about 179 degrees, preferably between about 10 degrees and about 40 degrees, and more preferably about 30 degrees. In some embodiments, the slit is between about 0,15 cm (0.06 inch), and about 2,54 cm (1.0 inch) in length, preferably between about 0,31 cm (0.12 inch) and about 1,95 cm (0.75 inch), and more preferably between about 0,38 cm (0.15 inch) and about 1,02 cm (0.40 inch). In some embodiments, the elongate catheter has a generally circular, oval, or rectangular cross-section and may have multiple lumens. In some embodiments having a second slit, the first and second slits are preferably symmetrical about a radial line and are arranged so as to converge at a point external to the elongate catheter. In other embodiments having a second slit, the first and second slits are asymmetrical about a radial line and are arranged so as to diverge from a point external to the elongate catheter.

In another aspect, the invention relates to a medical device including an elongate catheter including an external surface and at least one internal surface defining an internal lumen that extends longitudinally along at least a portion of the elongate catheter and a compound slit extending from the external surface to the at least one internal surface and into communication with the internal lumen. In some embodiments, the compound slit is disposed at a distal end of the catheter and the catheter slit valve includes a collar disposed at the distal end of the catheter.

In yet another aspect, the invention relates to a medical device including an elongate catheter defining a first lumen that extends longitudinally along at least a portion of the elongate catheter, a cap disposed at a distal end of the elongate catheter and including an external surface and at least one internal surface defining a second lumen, and a compound slit that extends from the external surface of the cap to the at least one internal surface of the cap and into communication with the first and second lumens.

In various embodiment of the two foregoing aspects, the compound slit is a tricuspid, T-shaped, cross-shaped, or double T-shaped. Also, the catheter and/or cap may include additional internal lumens, and may include a second compound slit.

In another aspect, the invention relates to a medical device that includes an elongate catheter defining an open distal end and a first lumen that extends longitudinally from the open distal along at least a portion of the elongate catheter, a cap disposed at the distal end of the elongate catheter, and a slit. The cap includes a proximal portion, an external surface, and at least one internal surface, and defines a second lumen and at least one slot in the proximal portion. The slit extends from the external surface of the cap to the at least one internal surface of the cap and into communication with the first and second lumens. In various embodiments, the slit is nonradial or a compound slit. Also, the open distal end of the catheter can be collapsible and the catheter has a wall thickness that tapers down in an area about the open distal end.

These and other objects, along with advantages and features of the present invention herein disclosed, will become apparent through reference to the following description, the accompanying drawings, and the claims. Furthermore, it is to be understood that the features of the various embodiments described herein are not mutually exclusive and can exist in various combinations and permutations.

### Brief Description of the Drawings

In the drawings, like reference characters generally refer to the same parts throughout the different views. Also, the drawings are not necessarily to scale, emphasis instead generally being placed upon illustrating the principles of the invention. In the following description, various embodiments of the present invention are described with reference to the following drawings in which:
- FIG. 1 is a side view, in partial cross-section, of a catheter in a vessel;
- FIG. 2B is a partial elevation of a catheter with a radial slit valve and FIG. 2A is a cross-sectional view of the catheter and slit valve of FIG. 2B taken at line 2A-2A;
- FIG. 3B is a partial elevation of one embodiment of a catheter with a linear nonradial slit valve and FIG. 3A is a cross-sectional view of the catheter and slit valve of FIG. 3B taken at line 3A-3A;
- FIG. 4A is a cross-sectional view of a catheter with a linear nonradial slit valve during infusion, and FIG. 4B is a cross-sectional view of a catheter with a linear radial slit valve during infusion;
- FIG. 5B is a partial elevation of one embodiment of a catheter with two linear nonradial slit valves and FIG. 5A is a cross-sectional view of the catheter and slit valves of FIG. 5B taken at line 5A-5A;
- FIG. 6B is a partial elevation of another embodiment of a catheter with two linear nonradial slit valves and FIG. 6A is a cross-sectional view of the catheter and slit valves of FIG. 6B taken at line 6A-6A;
- FIG. 7B is a partial elevation of one embodiment of a catheter with a nonlinear nonradial slit valve and FIG. 7A is a cross-sectional view of the catheter and slit valve of FIG. 7B taken at line taken at line 7A-7A;
- FIGS. 8B and 8C are partial elevations of alternate embodiments of a catheter with a nonlinear nonradial slit valve and FIG. 8A is a schematic cross-sectional view of the catheter and slit valve of FIG. 8B or 8C taken at line 8A-8A;
- FIG. 9B is a partial elevation of one embodiment of a catheter with two nonlinear nonradial slit valves and FIG. 9A is a cross-sectional view of the catheter and slit valves of FIG. 9B taken at line 9A-9A;
- FIG. 10B is a partial elevation of one embodiment of a catheter with two linear, offset, nonradial slit valves and FIG. 10A is a cross-sectional view of the catheter and slit valves of FIG. 10B taken at line 10A-10A;
- FIG. 11 is a cross-sectional view of one embodiment of a catheter with two lumens and two nonradial slit valves in each lumen;
- FIG. 12 is a cross-sectional view of another embodiment of a catheter with two lumens and two nonradial slit valves in each lumen;
- FIG. 13 is a cross-sectional view of yet another embodiment of a catheter with two lumens and two nonradial slit valves in each lumen;
- FIG. 14 is a cross-sectional view of one embodiment of a catheter with two offset lumens and two nonradial slit valves, one valve disposed in each lumen;
- FIGS. 15A-15C are cross-sectional views of a catheter with a nonradial slit valve during various stages of operation;
- FIG. 16B is a partial elevation of one embodiment of a catheter with a linear nonradial slit valve and a laminate disposed thereon and FIG. 16A is a cross-sectional view of the catheter and slit valve of FIG. 16B taken at line 16A-16A;
- FIG. 17B is a partial elevation of another embodiment of a catheter with a linear nonradial slit valve and a laminate disposed thereon and FIG. 17A is a cross-sectional view of the catheter and slit valve of FIG. 17B taken at line 17A-17A;
- FIG. 18B is a partial elevation of one embodiment of a catheter with a protuberance and a linear nonradial slit valve and FIG. 18A is a cross-sectional view of the catheter and slit valve of FIG. 18B taken at line 18A-18A;
- FIG. 19B is a partial elevation of another embodiment of a catheter with a protuberance and a linear nonradial slit valve and FIG. 19A is a cross-sectional view of the catheter and slit valve of FIG. 19B taken at line 19A-19A;
- FIG. 20B is a partial elevation of yet another embodiment of a catheter with a protuberance and a linear nonradial slit valve and FIG. 20A is a cross-sectional view of the catheter and slit valve of FIG. 20A taken at line 20A-20A;
- FIG. 21B is a partial elevation of still another embodiment of a catheter with a protuberance and a linear nonradial slit valve and FIG. 21A is a cross-sectional view of the catheter and slit valve of FIG. 21B taken at line 21A-21A;
- FIG. 22 is a schematic cross-sectional view of one embodiment of a catheter with two protuberances and two nonradial slit valves;
- FIG. 23 is a cross-sectional view of the catheter of FIG. 22 during infusion;
- FIG. 24 is a cross-sectional view of the catheter of FIG. 22 during aspiration;
- FIG. 25 is a cross-sectional view of an another embodiment of a catheter with two protuberances and two nonradial slit valves;
- FIG. 26 is a cross-sectional view of the catheter of FIG. 25 during infusion;
- FIG. 27 is a cross-sectional view of the catheter of FIG. 25 during aspiration;
- FIG. 28 is a longitudinal cross-sectional view of a catheter with two offset protuberances;
- FIGS. 29A-D are partial elevations of catheters with various compound slit valves;
- FIGS. 30A and 30B are a partial elevation and an end view of a catheter with a tricuspid slit valve;
- FIG. 31A is a partial elevation of one embodiment of a catheter and cap assembly with a linear nonradial slit valve located in the cap and FIG. 31B is a cross-sectional view of the catheter and cap assembly of FIG. 31A taken at line 31B-31B;
- FIG. 32A is a partial elevation of one embodiment of a catheter and cap assembly with a tricuspid slit valve located in the cap and FIG. 32B is a cross-sectional view of the catheter and cap assembly of FIG. 32A taken at line 32B-32B;
- FIGS. 33A and 33B are a partial elevation and an end view of one embodiment of a dual lumen catheter and cap assembly with two compound slit valves located in the cap;
- FIGS. 34A and 34B are a partial elevation and an end view of one embodiment of a dual lumen catheter with two compound slit valves;
- FIGS. 35A-C are partial elevations of various catheters and cap assemblies;
- FIGS. 36A and 36B are a partial elevation and an end view of a catheter and cap assembly with a slotted cap and a nonlinear nonradial slit valve located in the cap; and
- FIGS. 36C and 36D are cross-sections of the catheter and cap assembly shown in FIGS 36A and 36B taken at lines 36C-36C and 36D-36D, respectively.

### Description

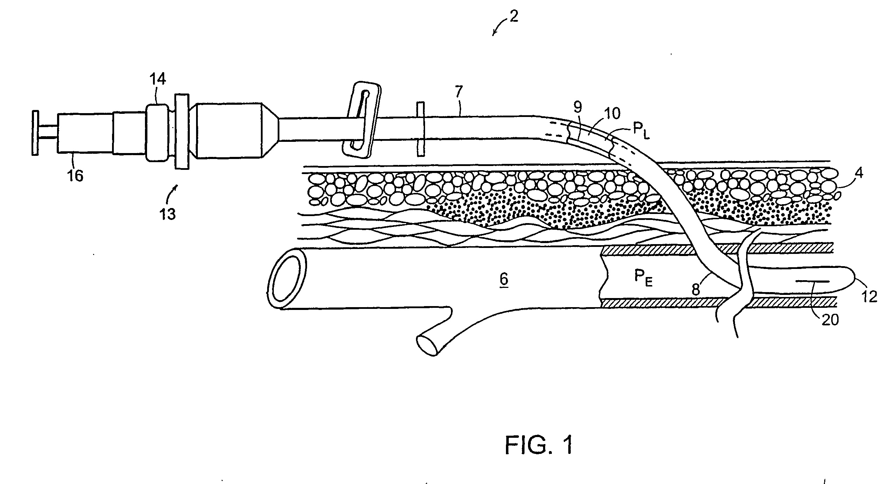

Referring to FIG. 1, a catheter 2 is placed through and/or beneath the skin 4 and into a vessel 6 of a patient for either infusing a fluid (such as a drug, nutrient, blood, or other body fluid) into the body and/or aspirating a fluid from the body. The catheter 2 includes an elongated member 7 (of a biocompatible material, such as a polymeric material) that has an external surface 8 exposed to the body environment and an internal surface 9 defining a lumen 10, the lumen 10 extends longitudinally along at least a portion of the catheter 2. Various permutations of the catheter 2 will be described hereinbelow with respect to various aspects of the invention and accompanying figures. The lumen 10 is closed at the distal end 12 of the catheter 2 and can be accessed at the proximal end 13 through a fitting 14, for example a standard luer lock, which is connected to a syringe 16 or other suitable device for injecting or withdrawing fluid from the lumen 10.

The portions of the catheter 2 that is inserted into the vessel 6 has a slit valve 20, which permits fluid communication between the body environment and the lumen 10 by varying the pressure in the lumen (P_{L}) relative to the pressure in the body environment (P_{E}).

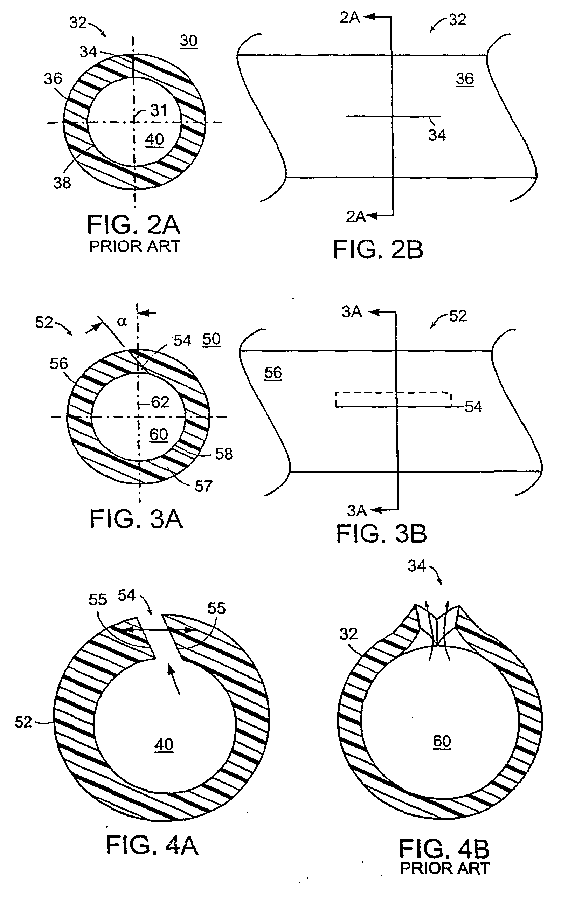

FIGS. 2A and 2B illustrate a known medical device 30 that includes a catheter 32 (for simplicity, only a portion of the catheter is shown) with a radial slit valve 34. The catheter 32 has an external surface 36 and an internal surface 38 and defines a lumen 40. As can be seen, the slit 34 extends radially between the external surface 36 and the internal surface 38. In other words, a line passing through the slit 34 would pass through the center point 31 of the lumen 40.

FIGS. 3A and 3B illustrate a medical device 50 that includes a catheter 52 and a nonradial slit valve 54. The catheter 52 has an external surface 56 and an internal surface 58 and defines a lumen 60. The external surface 56 and internal surface 58 define the catheter wall 57. The wall thickness will vary as necessary depending on the application, e.g., where the catheter will be used, the overall size of the catheter, the pressures the catheter will be exposed to, and the material stiffness required. Generally, the wall thickness should be such that the catheter 52 remains flexible enough to allow the slit valve 54 to function properly, but is stiff enough to resist the unintentional influx of blood in to the lumen(s). In addition, the wall thickness may vary about the catheter diameter and/or along the length of the catheter. Further, the catheter 52 has a generally circular cross-section; however, the cross-section could be any suitable shape, for example elliptical, rectangular, or oval.

The slit valve 54 is linear and generally longitudinally disposed on the catheter 52. The slit 54 extends nonradially between the external surface 56 and the internal surface 58. The slit 54 facilitates communication between the lumen 60 and an environment external to the catheter 52 by opening and closing in response to pressure changes in either the lumen or the environment or both. As discussed above, the nonradial slit 54 allows the valve to flex more easily than a conventional radial slit as shown in FIG. 2A. The nonradial slit 54 intersects with a radial line 62 to form an included angle (α). The angle α shown in FIG. 3A is approximately 45 degrees; however, α could range from about 1 degree to about 179 degrees in any of the embodiments. Furthermore, the slit valve 54 is preferably disposed on a distal portion of the catheter 52 to reduce dead space at the distal end of the catheter, where blood or other fluids may collect; however, the slit valve 54 may be located at any location along the catheter 52.

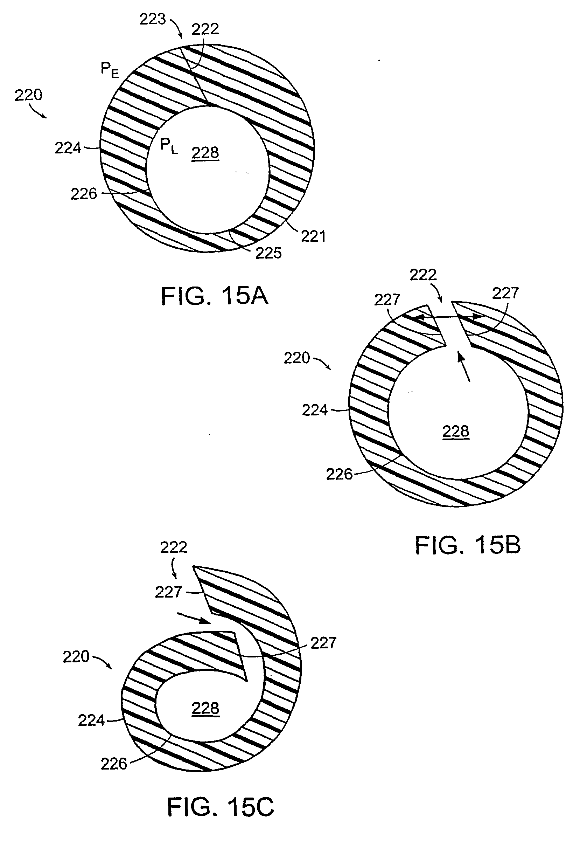

FIG. 4A depicts the catheter 52 of FIG. 3A during infusion. FIG. 4B depicts the catheter 32 of FIG. 2A during infusion. As can be seen, the walls 55 of the nonradial slit valve move apart for infusion of fluids as opposed to moving outwardly, as occurs with the radial slit valve 34. Therefore, nonradial slit valve 54 has a greater opening during operation than the radial slit valve 34. A further discussion of the operation of various embodiments of the nonradial slit valves can be found hereinbelow with respect to FIGS 15A-C and 22-27.

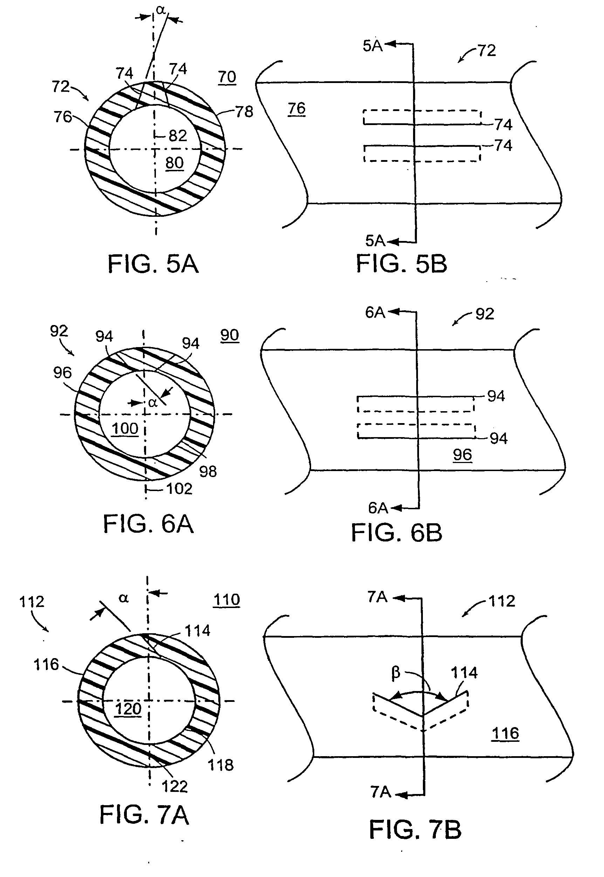

FIGS. 5A and 5B illustrate a medical device 70 that includes a catheter 72 and two nonradial slit valves 74. The catheter 72 has an external surface 76 and an internal surface 78 and defines a lumen 80. The slit valves 74 are linear and generally longitudinally disposed on the catheter 72. The slits 74 extend nonradially between the external surface 76 and the internal surface 78. The slits 74 facilitate communication between the lumen 80 and an environment external to the catheter 72. As discussed with respect to FIGS. 3A and 3B, each nonradial slit 74 is oriented such that a line passing through the slit 74 would intersect with radial line 82 to form an included angle (α). The angle α shown in FIG. 5A is approximately 30 degrees. The slits 74 may be disposed symmetrically about radial line 82 and oriented such that lines passing through the slits 74 would converge at a point external to the catheter 72. In any of the embodiments described herein, the circumferential spacing between the slit valves can be varied as necessary. In addition, the medical device is not limited to two slit valves, but may have as many valves as is practical.

FIGS. 6A and 6B illustrate a medical device 90 that includes a catheter 92 and two nonradial slit valves 94. The catheter 92 has an external surface 96 and an internal surface 98 and defines a lumen 100. The slit valves 94 are linear and generally longitudinally disposed on the catheter 92. The slits 94 extend nonradially between the external surface 96 and the internal surface 98. The slits 94 facilitate communication between the lumen 100 and an environment external to the catheter 92. As discussed above, each nonradial slit 94 is oriented such that a line passing through the slit 94 would intersect a radial line 102 to form an included angle (α). The angle α shown in FIG. 6A is approximately 45 degrees. The slits 94 may be disposed symmetrically about radial line 102 and oriented such that lines passing therethrough would diverge from a point external to the catheter 92.

FIGS. 7A and 7B illustrate a medical device 110 that includes a catheter 112 and a nonradial slit valve 114. The catheter 112 has an external surface 116 and an internal surface 118 and defines a lumen 120. The slit valve 114 is nonlinear and generally longitudinally disposed on the catheter 112. The slit valve 114 includes an angle (β). The angle β shown is approximately 120 degrees; however, β could be any angle from about 45 degrees to about 179 degrees. Also, the slit valve 114 may include more than one angle. The slit 114 extends nonradially between the external surface 116 and the internal surface 118. The slit 114 facilitates communication between the lumen 120 and an environment external to the catheter 112. As discussed above, the nonradial slit 114 intersects a radial line 122 to form an included angle (α). The angle α shown is approximately 45 degrees.

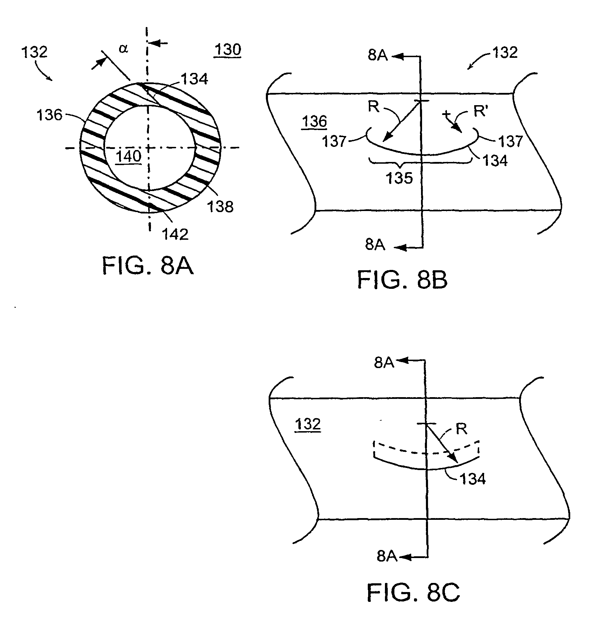

FIGS. 8A-8C illustrate a medical device 130 that includes a catheter 132 and a nonradial slit valve 134. The catheter 132 has an external surface 136 and an internal surface 138 and defines a lumen 140. The slit valve 134 is nonlinear and generally longitudinally disposed on the catheter 132. The slit valve 132 depicted in FIG. 8B includes a major curved portion 135 having a radius (R) and two smaller curved portions 137, each having a radius (R') and disposed at an end of the slit valve 134. Alternatively the slit valve 134 may include only a single curve, as shown in FIG. 8C. The slit 134 extends nonradially between the external surface 136 and the internal surface 138. The slit 134 facilitates communication between the lumen 140 and an environment external to the catheter 132. As discussed above, the nonradial slit 134 intersects a radial line 142 to form an included angle (α). The angle α shown is approximately 45 degrees.

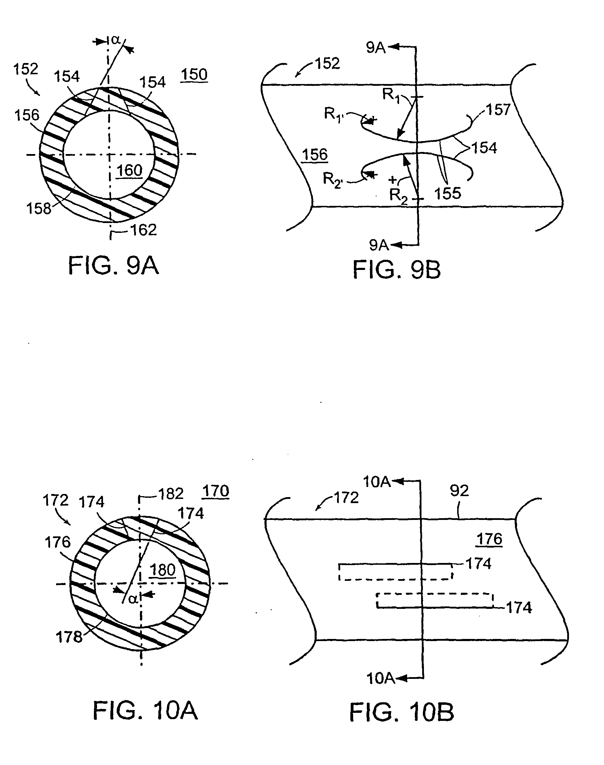

FIGS. 9A and 9B illustrate a medical device 150 that includes a catheter 152 and two nonradial slit valves 154. The catheter 152 has an external surface 156 and an internal surface 158 and defines a lumen 160. The slit valves 154 are nonlinear and generally longitudinally disposed on the catheter 152. The slit valves 154 each include a major curved portion 155 having a radius (R1, R2) and two smaller curved portions 157, each having a radius (R₁', R₂'), disposed at the ends of the slit valve 154. Alternatively, each slit valve 154 may include only a single curve. In the embodiment shown, R₁ and R₂ and R₁' and R₂' are approximately equal; however, the dimensions of the slit valves 154 can be varied to suit a particular application. The slits 154 extend nonradially between the external surface 156 and the internal surface 158. The slits 154 facilitate communication between the lumen 160 and an environment external to the catheter 152. As discussed above, each nonradial slit 154 is oriented such that a line passing through the slit 154 would intersect a radial line 162 to form an included angle (α). The angle α shown is approximately 30 degrees. The slits 154 may be disposed symmetrically about radial line 162 and oriented such that lines passing therethrough would converge at a point external to the catheter 152. Alternatively, the slits 154 may be oriented such that lines passing therethrough would diverge from a point external to the catheter 152

FIGS. 10A and 10B illustrate a medical device 170 that includes a catheter 172 and two nonradial slit valves 174. The catheter 172 has an external surface 176 and an internal surface 178 and defines a lumen 180. The slit valves 174 are linear and generally longitudinally disposed on the catheter 172. In addition, the slit valves 174 are longitudinally offset. The slits 174 extend nonradially between the external surface 176 and the internal surface 178. The slits 174 facilitate communication between the lumen 180 and an environment external to the catheter 172. As discussed above, each nonradial slit 174 is oriented such that a line passing through the slit 174 would intersect with radial line 182 to form an included angle (α). The angle α shown is approximately 45 degrees. The slits 174 may be disposed symmetrically about radial line 182 and oriented such that lines passing therethrough would diverge from a point external to the catheter 172. Alternatively, the slits 174 may be oriented such that lines passing through the slits 174 would converge at a point external to the catheter 172.

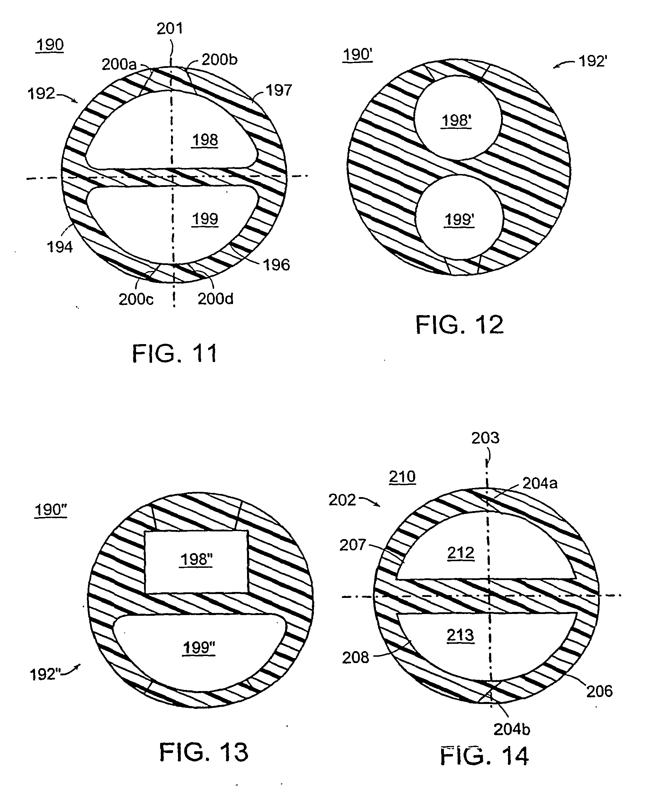

FIG. 11 illustrates a medical device 190 in cross-section. The medical device 190 includes a catheter 192 and four nonradial slit valves 200. The catheter 192 has an external surface 194 and two internal surfaces 196, 197. Each internal surface 196, 197 defines a lumen 198, 199. The lumens 198, 199 are shown having a "D" shape; however, each lumen 198, 199 can have any suitable shape. For example, FIG. 12 depicts a catheter 192' having two circular lumens 198', 199', and FIG. 13 depicts a catheter 192" having a rectangular lumen 198" and a "D" shaped lumen 199". In addition, the catheter 192 is not limited to two lumens 198, 199, and may have as many lumens as is practical.

The slit valves 200 are generally longitudinally disposed on the catheter 192 and may be linear or nonlinear. Two slits 200a,b extend nonradially between external surface 194 and internal surface 197 and two slits 200c,d extend nonradially between external surface 194 and internal surface 198. The slits 200 facilitate communication between the lumens 198, 199 and an environment external to the catheter 192. As discussed above, each nonradial slit 200 is oriented such that a line passing through the slit 200 would intersect a radial line 201 to form an included angle (α). Slits 200a,b are disposed symmetrically about radial line 201 and are oriented such that lines passing through slits 200a,b would converge at a point external to the catheter 192. Slits 200c,d are disposed symmetrically about radial line 201 and are oriented such that lines passing through slits 200c,d would diverge from a point external to the catheter 192.

FIG. 14 illustrates a medical device 210 in cross-section. The medical device 210 includes a catheter 202 and two nonradial slit valves 204. The catheter 202 has an external surface 206 and two internal surfaces 207, 208. Each internal surface 207, 208 defines a generally D-shaped offset lumen 212, 213. Essentially, the lumens 212, 213 are offset to form a thickened wall portion for the slits 204. The slit valves 204 are longitudinally disposed on the catheter 202 and may be linear or nonlinear. Slit 204a extends nonradially between the external surface 206 and internal surface 208 and slit 200b extend nonradially between the external surface 206 and internal surface 207. The slits 204 are disposed in the area of the catheter having a thickened wall portion. The slits 204 facilitate communication between the lumens 212,213 and an environment external to the catheter 202. As discussed above, each nonradial slit 204 is oriented such that a line passing through the slit 204 would intersect a radial line 203 to form an included angle (α). It should be noted that the medical devices described herein are not limited to any specific number or combination of lumens and slit valves, but may have any number or combination as is practical for a specific application.

FIGS. 15A-15C depict a medical device 220 including a catheter 221 with a nonradial slit valve 222 during various stages of operation. The catheter 221 has an external surface 224 and an internal surface 226 and defines a lumen 228. The catheter 221 has a generally circular cross-section and the lumen 228 is eccentric with respect to the external surface 224. Generally, a catheter slit valve with an eccentric lumen is easier to manufacture, because fewer steps are required. For example, no chemical weakening of the catheter wall opposite or adjacent the slit is required. Also, the combination of a nonradial slit and an eccentric lumen results in a larger aperture for aspiration. The slit valve 222 is located in the area of the greatest wall thickness (thickened wall portion 223) and is consistent with the various embodiments of slit valves previously discussed. The area of the catheter 221 opposite the thickened wall portion 223 has the thinnest wall (thinned wall portion 225). The thinned wall portion 225 acts like a hinge during infusion.

In FIG. 15A, the slit valve 222 is shown in the sealed condition, where the lumen pressure P_{L} is essentially equal to the environmental pressure P_{E}. FIG. 15B depicts the slit valve 222 in infusion mode. The pressure within the lumen 228 is increased with respect to the environmental pressure, thereby causing the catheter walls 227 adjacent the slit 222 to begin to move apart, allowing infusion of fluid to the external environment. FIG. 15C depicts the slit valve 222 in aspiration mode. As the lumen pressure decrease with respect to the environmental pressure, one side of the slit valve 222 yields, or flexes inwardly, to allow fluid to enter the catheter lumen 228. More specifically, as the lumen pressure decreases, a change in the catheter cross-section occurs involving a large portion of the wall, which deflects as a result of the decreased internal pressure. The geometry of the lumen 228 and nonradial slit 222 results in an improved aspiration function with respect to conventional radial slit valves (see FIGS. 2A and 2B). The improved function is defined, in part, by a reduced pressure required to initially open the valve 222, improved volume flow, and a reduction of valve sticking, which can occur in conventional radial slit valves.

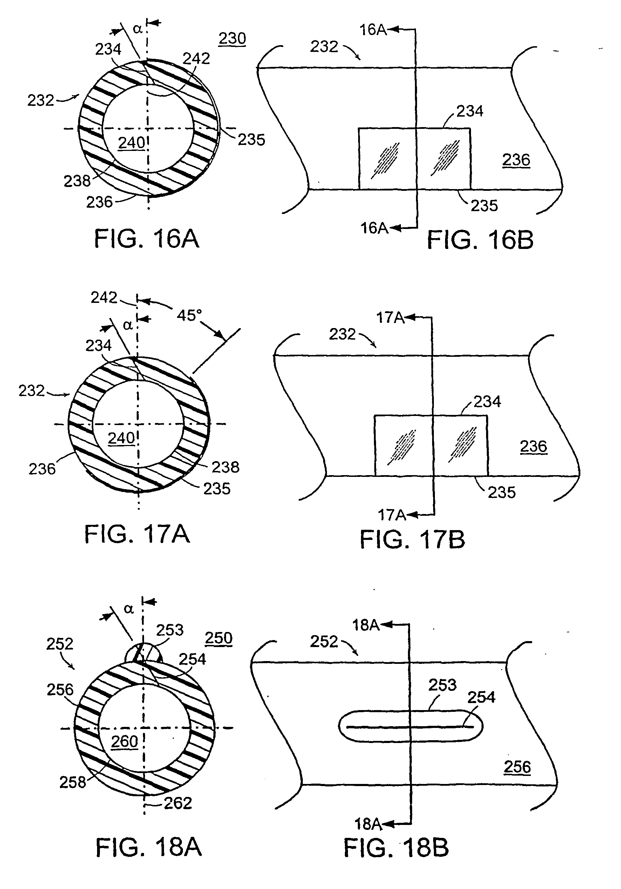

FIGS. 16A and 16B illustrate a medical device 230 that includes a catheter 232 and a nonradial slit valve 234. The catheter 232 has an external surface 236 and an internal surface 238 and defines a lumen 240. The slit valve 234 is linear and generally longitudinally disposed on the catheter 232. The slit 234 extends nonradially between the external surface 236 and the internal surface 238 and facilitates communication between the lumen 240 and an environment external to the catheter 232. The nonradial slit 234 intersects with a radial line 242 to form an included angle (α). The angle α shown is approximately 45 degrees. The medical device 230 further includes a laminate 235 disposed on the external surface 236. The laminate 235 begins at the slit 234 and extends up to about 225 degrees about the external surface 236. In an alternative embodiment shown in FIGS. 17A and 17B, the laminate 235 begins about 45 degrees from the slit 234 and extends up to about 225 degrees from the slit 234. The laminate 235 stiffens the catheter wall to facilitate the operation of the slit valve. Specifically, the non-laminated side of the slit 234 is more flexible than the laminated side and should cause the non-laminated side to yield reliably with respect to the laminated side. In this particular embodiment, it is preferred that the nonradial slit be cut towards the laminated side. The laminate 235 may be constructed of the same material as the catheter 232, for example polyurethane or silicone, but preferably with a higher durometer. Catheter materials are discussed in greater detail hereinbelow. The laminate 235 may be attached to the catheter 232 by adhesive bonding, solvent bonding, or similar technique. Generally, solvent bonding includes using a solvent to facilitate fusing of the laminate 235 with the catheter 232.

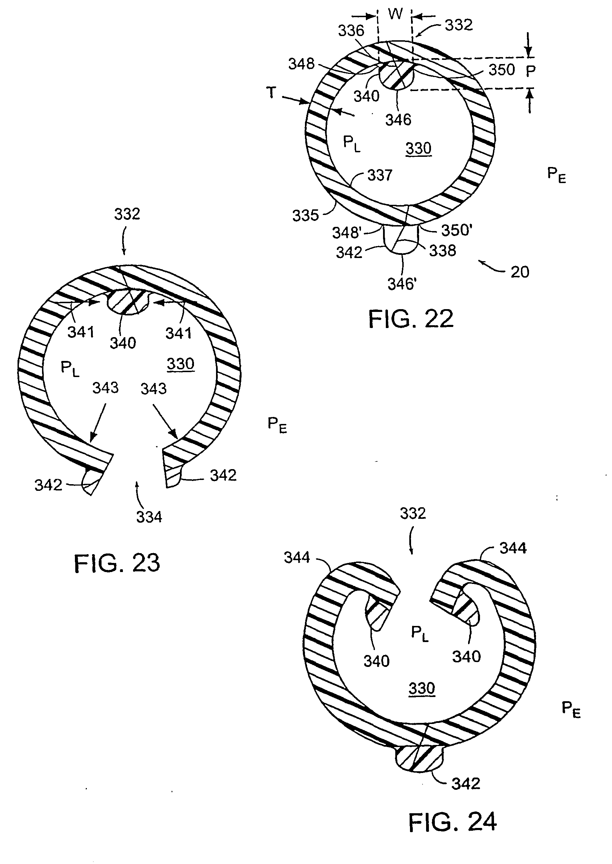

FIGS. 18A and 18B illustrate a medical device 250 that includes a catheter 252, a protuberance 253, and a nonradial slit valve 254. The catheter 252 has an external surface 256 and an internal surface 258 and defines a lumen 260. The protuberance 253 is disposed on the external surface 256 of the catheter 252. The function of the protuberance 253 is discussed hereinafter with respect to FIGS. 22-27. The size and shape of the protuberance 253 may vary and is described in greater detail with respect to FIG. 22. The slit valve 254 extends through the protuberance 253 and both are linear and generally longitudinally disposed on the catheter 252. The slit 254 extends nonradially from the external surface 256, through the protuberance 253, and to the internal surface 258. The slit 254 facilitates communication between the lumen 260 and an environment external to the catheter 252. The nonradial slit 254 intersects with a radial line 262 to form an included angle (α). The angle α shown is approximately 45 degrees.

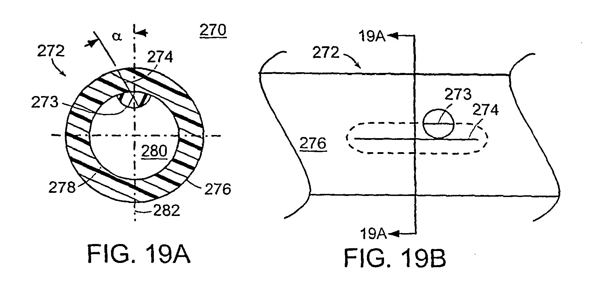

FIGS. 19A and 19B illustrate a medical device 270 that includes a catheter 272, a protuberance 273, and a nonradial slit valve 274. The catheter 272 has an external surface 276 and an internal surface 278 and defines a lumen 280. The protuberance 273 is disposed on the internal surface of the catheter. The function of the protuberance 273 is discussed hereinafter with respect to FIGS. 22-27. As discussed above, the size and shape of the protuberance may vary. The slit valve 274 extends through the protuberance 273 and both are linear and generally longitudinally disposed on the catheter 272. The slit 274 extends nonradially between the external surface 276 and the internal surface 278 and facilitates communication between the lumen 280 and an environment external to the catheter 272. The nonradial slit 274 intersects with a radial line 282 to form an included angle (α). The angle α shown is approximately 45 degrees.

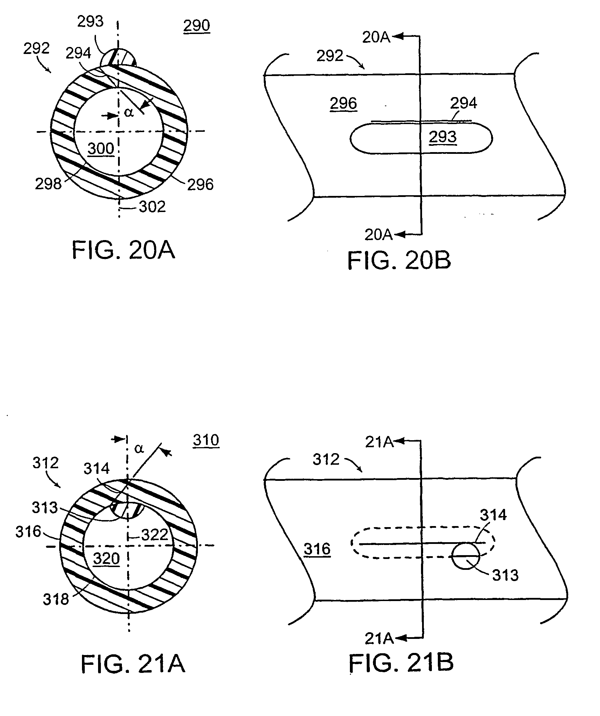

FIGS. 20A and 20B illustrate a medical device 290 that includes a catheter 292, a protuberance 293, and a nonradial slit valve 294. The catheter 292 has an external surface 296 and an internal surface 298 and defines a lumen 300. The protuberance 293 is disposed on the external surface 296 of the catheter 292 and acts to reinforce one side of the slit valve 294, similar to the laminate 235 of FIGS. 16A and 16B. As discussed above, the size and shape of the protuberance may vary. The slit valve 294 is disposed adjacent to the protuberance 293 and both are linear and generally longitudinally disposed on the catheter 292. The slit 294 extends nonradially between the external surface 296 and the internal surface 298 and facilitates communication between the lumen 300 and an environment external to the catheter 292. The nonradial slit 294 intersects with a radial line 302 to form an included angle (α). The angle α shown is approximately 45 degrees.

FIGS. 21A and 21B illustrate a medical device 310 that includes a catheter 312, a protuberance 313, and a nonradial slit valve 314. The catheter 312 has an external surface 316 and an internal surface 318 and defines a lumen 320. The protuberance 313 is disposed on the internal surface 318 of the catheter 312. The protuberance 313 acts to stiffen one side of the valve 314, and has a similar effect as the laminate 235 discussed hereinabove. As discussed above, the size and shape of the protuberance may vary. The slit valve 314 is disposed adjacent to the protuberance 313 and both are linear and generally longitudinally disposed on the catheter 312. The slit 314 extends nonradially between the external surface 316 and the internal surface 318 and facilitates communication between the lumen 320 and an environment external to the catheter 312. The nonradial slit 314 intersects with a radial line 322 to form an included angle (α). The angle α shown is approximately 45 degrees.

Figs. 22-24, illustrate a medical device including a catheter 328 and a pair of slit valves 332, 334 in a series of cross-sectional views. In this embodiment, the catheter cross-section is circular; however, the cross-section could be any suitable shape, for example elliptical, rectangular, or oval. In FIG. 22, the valves 332, 334 are shown in the sealed condition, where the lumen pressure P_{L} is essentially equal to the environmental pressure P_{E}. The first valve 332 is used only for aspiration. The second valve 334 is used only for infusion. Each valve 332, 334 includes a nonradial slit 336, 338. The slits 336, 338 may be linear or nonlinear.

The valves 332, 334 also include protuberances 340, 342 that project from the catheter 328 and are disposed opposite each other. In this case, both valves 332, 334 have the same generally convex external surface 335, with the protuberances 340, 342 arranged to make the slit valves 332, 334 function as one-way valves in opposite directions; one one-way valve is for aspiration only and the other is for infusion only. The first slit valve 332 has a protuberance 340 projecting from the internal surface 337 into the lumen 330 of the catheter 328. Slit 336 extends through protuberance 340. The second valve 334 has a protuberance 342 projecting from the external surface 335 outward. Slit 338 extends through protuberance 342. As previously discussed, the slits 336, 338 extend nonradially between the external surface and the internal surface and intersect with a radial line to form an included angle (α). Also as previously discussed, the protuberances 340, 342 and slits 336, 338 may be disposed symmetrically about a radial line and oriented such that lines passing through the slits 336, 338 would diverge from a point external to the catheter 328 or converge at a point external to the catheter 328.

Referring to FIG. 23, the pressure in the lumen 330 is increased, for example by depressing the plunger of the syringe 16 (see FIG. 1), which creates a condition where the lumen pressure P_{L} is greater than the environment pressure P_{E}. The increased lumen pressure acts upon the inwardly projecting protuberance 340 to create a greater sealing force (arrows 341) than in the absence of the protuberance 340. The secure seal prevents any uncontrolled infusion through the first slit valve 332. In the second valve 334, the increased lumen pressure creates a force (arrows 343) that causes the catheter wall to flex outwardly along a flexure region spaced from the slit 338. The protuberance 342 does not substantially inhibit the opening of the slit valve, thereby permitting a controlled infusion to take place.

Referring to FIG. 24, the pressure within the lumen 330 is reduced by, for example, withdrawing the plunger of the syringe 16 (see FIG. 1). In the first valve 332, the reduced pressure causes the catheter wall to flex inwardly along flexure regions 344. The protuberance 340 does not substantially interfere with the inward flexing of the valve, thereby permitting controlled aspiration of fluid through the valve 332. In the second valve 334, the protuberance 342 resists inversion or collapse, thereby preventing uncontrolled aspiration through the second valve 334.

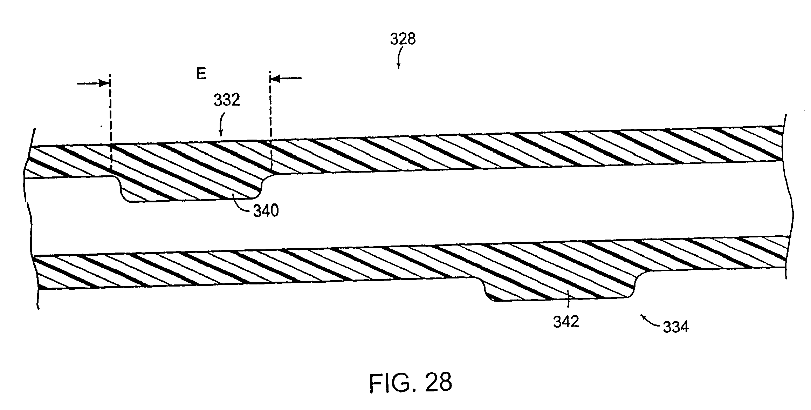

The protuberances 340, 342 assist valve operation by projecting into the environment or lumen 330. For example, projection of a protuberance into a lumen increases sealing forces on the slit when the pressure in the lumen is increased, because the protuberance modifies the contour about the slit such that the components of pressure vectors perpendicular to the slit are larger. The protuberances 340, 342 illustrated in FIGS. 22-24 are generally hemispherical in shape with geometrical inflections 346, 346' at the location of greatest projection and further geometrical inflections 348, 348' and 350, 350' near or at the boundaries of the protuberances 340, 342, where projection from the catheter 328 begins. Referring to FIG. 28, it can be seen that these protuberances 340, 342 also have short axial projections (L) that generally correspond to the length of the slit 336, 338.

The shape and dimensions of a protuberance may vary. The width of the protuberance (W) is preferably about twice the thickness (T) of the catheter body adjacent the protuberance or less. The length (L) generally corresponds to the length of the slit, but may be significantly greater in length than the slit and may act to stiffen the catheter. The projection of a protuberance (P) into a lumen is preferably equal to or less than the lumen diameter. The protuberance may project across substantially the entire width of a lumen. The projection from the outer surface of a catheter is preferably equal to or less than the outer diameter of the catheter. The protuberance may project from a catheter body having an otherwise uniform geometrical configuration, as indicated above, or the protuberance may project from a catheter body having an irregular or contoured inner and/or outer wall surface. The inflection points on either side of the protuberance are preferably spaced from the flexure or hinge region of the valve. The protuberance may also be asymmetrical in cross-section and may not have inflections at its boundaries, but instead extend smoothly from the otherwise uniform thickness and profile of the catheter wall. The protuberance may be oblong in shape.

One advantage of the embodiment shown in FIGS. 22-24 is that the catheter 328 may be operated at higher lumen pressures during infusion without inversion of the first valve 332, which could lead to leaks or uncontrolled infusion. Similarly, the outwardly extending protuberance 342 of the second valve 334 enhances sealing during aspiration through the first valve 332. Higher vacuum can be used during aspiration without inversion of the second valve 334. Alternatively, the catheter 328 can be arranged for operation at lower pressure differentials for both infusion and aspiration. For example, the body of the catheter 328 can be made of a weakened or thin-walled construction. The protuberance 342 of the second valve 334 prevents collapse of the second valve 334 during aspiration. During infusion through the second valve 334, the protuberance 340 of the first valve 332 prevents outward inversion. Further, a protuberance can be used to make a one-way valve from a valve that would ordinarily operate for both infusion and aspiration. For example, the valve wall may be weakened by chemical treatment or by reducing the thickness of the polymer, which would ordinarily encourage valve action in either direction; however, a protuberance may be used to prevent valve action in one direction.

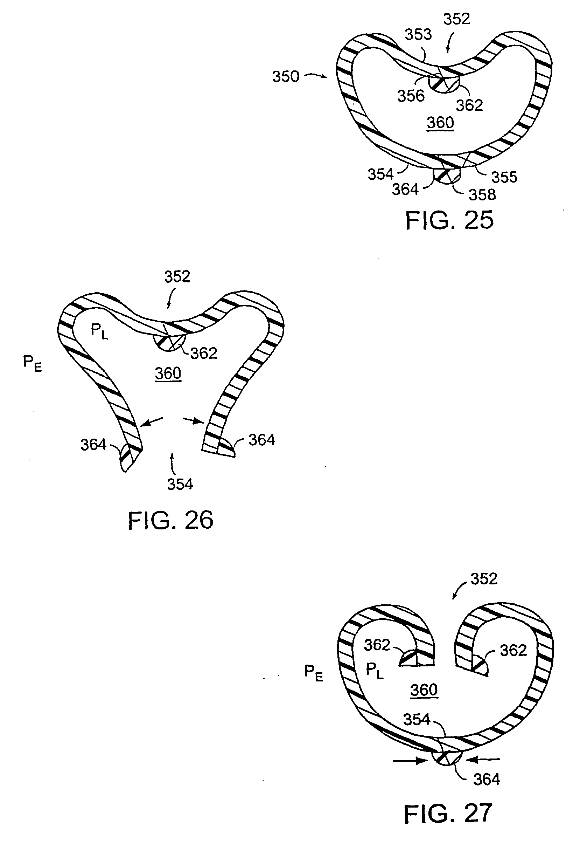

FIGS. 25-27 illustrate an alternative embodiment of the medical device of FIG. 22. The medical device includes a catheter 350 and a pair of slit valves 352, 3 54 in a series of cross-sectional views. In this embodiment, the first valve 352 is used for aspiration. It includes an external surface 353 that is generally concave and a slit 356 through the external surface 353 of the concave region. The concave surface assists opening of the slit 356 by inward flexing of the catheter wall when a low pressure condition exists in the lumen 360, thus facilitating aspiration. The second valve 354 has a generally convex external surface 355. The convex surface assists opening of the slit 358 by outward flexing of the catheter wall when pressure is increased in the lumen 360, which facilitates infusion.

Both valves 352, 354 include a protuberance 362, 364. In the first valve 352, the protuberance 362 projects into the lumen 360. In the second valve 354, the protuberance 364 projects radially outward into the environment. Slit 356 extends through protuberance 362 and slit 358 extends through protuberance 364.

During infusion, the inwardly projecting protuberance 362 enhances sealing of the first valve 352 (FIG. 26) when lumen pressure is increased to open the second valve 354. During aspiration, fluid is aspirated through the first valve 352 (FIG. 27), without interference from the protuberance 362, while sealing is enhanced at the second valve 354 by the outward projecting protuberance 364. The protuberance 362 on the infusion valve prevents inversion at the somewhat higher pressure differentials needed for operation of an aspiration valve with a convex outer surface.

Alternatively, both valves 352, 354 could have concave outer surfaces 353, with the first valve 352 having an inwardly projecting protuberance 362 and the second valve 354 having an outwardly projecting protuberance 364. The first valve 352 would be used for aspiration and the second valve 354 would be used for infusion.

In yet another alternative embodiment, both the first and second valves 352, 354 could include outwardly projecting protuberances and/or protuberances of unequal dimensions. For example, the first valve protuberance 362 projects less than the second valve protuberance 364. For aspiration, a controlled lumen pressure causes the first valve 352 to invert while the larger protuberance 364 in the second valve 354 prevents inversion. For infusion, an increase in pressure causes both first and second valves 352, 354 to open.

FIG. 28 is a longitudinal cross-section of the embodiment depicted in FIGS. 22-24. The catheter 328 has a first valve 332 that includes an inwardly projecting protuberance 340 and a second valve 334 that includes an outwardly projecting protuberance 342. The valves 332, 334 and protuberances 340, 342 are disposed opposite each other. In addition, the valves 332, 334 and protuberances 340, 342 are longitudinally offset.

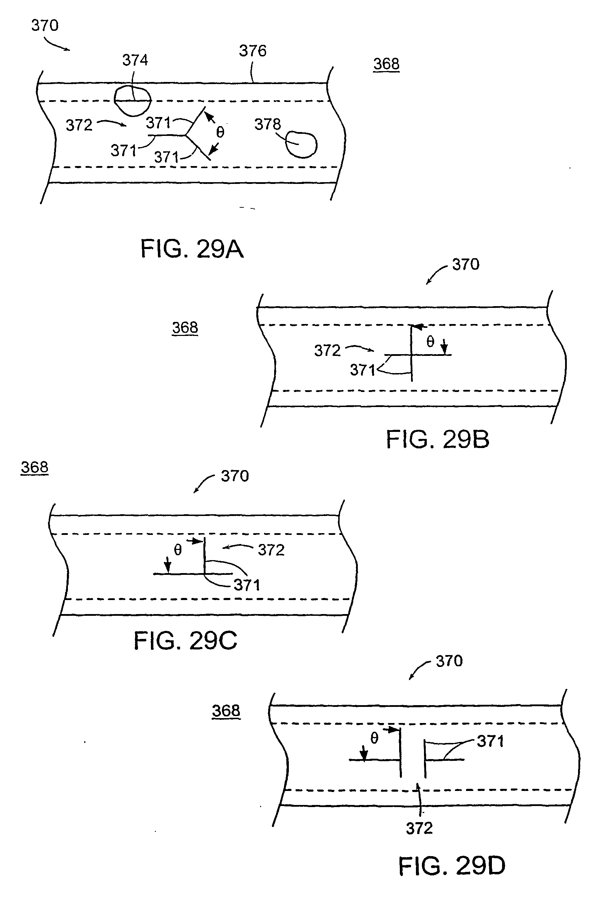

FIG. 29A illustrates a medical device 368 that includes a catheter 370 and a compound slit valve 372. The catheter 370 has an internal surface 374 and an external surface 376 and defines a lumen 378. The compound slit valve 372 is tricuspid in shape and includes three generally linear slits 371 disposed on the catheter 370 and extending between the external surface 376 and the internal surface 374. The slits 371 facilitate communication between the lumen 378 and an environment external to the catheter 370. The slits 371 extend outwardly from a common origin and are equiangularly spaced, i.e., θ equals 120 degrees. However, the value of θ may vary from about 90 degrees to about 180 degrees. In alternative embodiments, the catheter 370 may include multiple compound slit valves 372 or multiple lumens 378 or both. In addition, the catheter 370 may include a combination of compound slit valves and nonradial slit valves.

Fig. 29B illustrates a medical device 368 that has a cross-shaped compound slit valve 272. Fig. 29C illustrates a medical device 368 that has a T-shaped compound slit valve 272. Fig. 29D illustrates a medical device 368 that has a double T-shaped compound slit valve 272. The compound slit valve 372 can be oriented in any direction on the catheter 370, and can be disposed on a distal end of the catheter 370. As discussed with respect to FIG. 29A, the compound slit valve 372 includes a plurality of intersecting slits 371. The slits 371 shown in FIGS. 29B-29D intersect at right angles, i.e., θ equals 90 degrees; however, the value θ of may vary from about 1 degree to about 179 degrees.

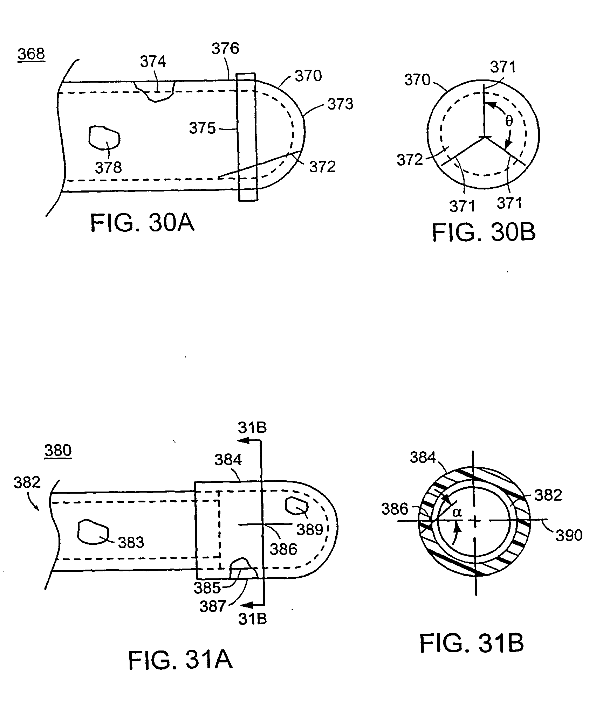

FIGS. 30A and 30B illustrate an alternative embodiment of the medical device 368 of FIG. 29A. The medical device 368 includes a catheter 370 and a tricuspid slit valve 372 disposed on a forward-facing distal tip 373 of the catheter 370. In this embodiment, the tip 373 has a generally convex, hemispherical outer surface. As depicted in FIG. 29A, the catheter 370 has an internal surface 374 and an external surface 376 and defines a lumen 378. The slit valve 372 includes three generally linear slits 371 extending between the external surface 376 and the internal surface 374. The slits 371 extend outwardly from a common origin and are equiangularly spaced, i.e., θ equals 120 degrees. Also in this embodiment, the catheter 370 includes a collar 375 disposed about the distal tip 373. The collar 375 helps to stiffen the valve 372 to reduce the possibility of leakage. Furthermore, a catheter 370 with a slit valve 372 at the distal tip 373 may make placement of the medical device 368 over a guidewire (not shown) easier.

FIGS. 31A and 31B illustrate a medical device 380 that includes a catheter 382 and a cap 384 with a nonradial slit valve 356. The catheter 382 is essentially the same as any of the catheters described herein, e.g., the catheter 382 defines a lumen 383. The cap 384 has an external surface 387 and an internal surface 385 and also defines a lumen 389. The slit valve 386 depicted is linear and generally longitudinally disposed on the cap 384; however, as discussed hereinabove, the slit valve could be nonlinear. The slit 386 extends nonradially between the external surface 387 and the internal surface 385 of the cap 384. The slit 386 facilitates communication between an environment external to the catheter 382 and lumens 383, 389. As discussed with respect to FIG. 3, the nonradial slit 386 is oriented such that a line passing through the slit 386 would intersect with radial line 390 to form an included angle (α).

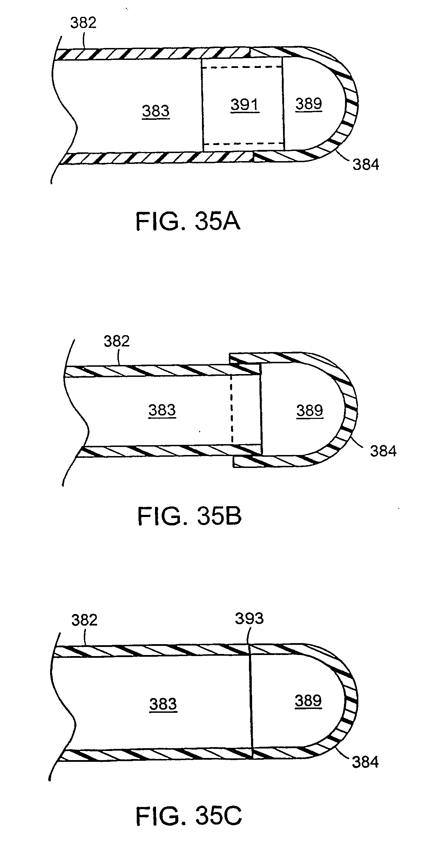

The cap 384 can be coupled to the catheter 382 by chemical or adhesive bonding or the cap 384 may be sized such that it is force fit over the catheter 382 and held in place by frictional force. The cap 384 and catheter 382 are depicted as coupled in an overlapping fashion; however, the cap 384 and catheter 382 may be coupled end to end. See FIGS. 35A-C for illustrations of various catheter and cap assemblies.

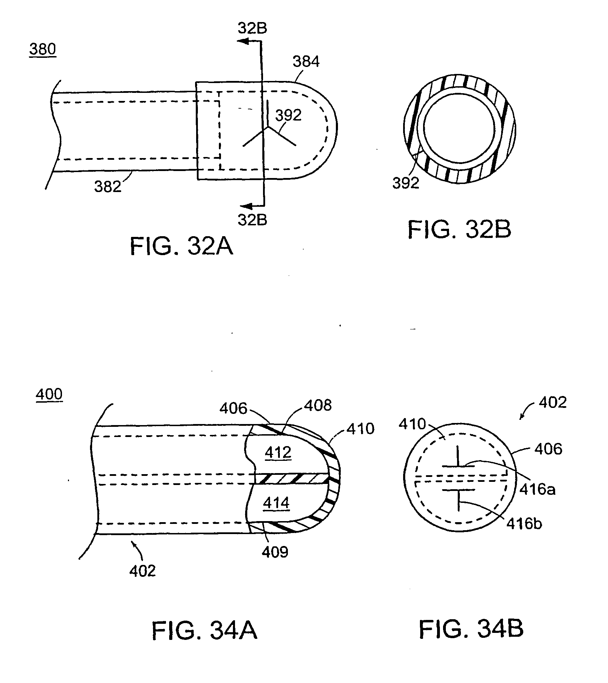

FIGS. 32A and 32B depict an alternative embodiment of the medical device 380 of FIG. 31A and 31B. The catheter 382 is essentially the same as any of the catheters described herein, e.g., the catheter 382 defines a lumen 383. The cap 384 is essentially the same as the cap described with respect to FIGS. 31A and 31B; however, the cap 384 has a tricuspid slit valve 392 as opposed to a nonradial slit valve 386.

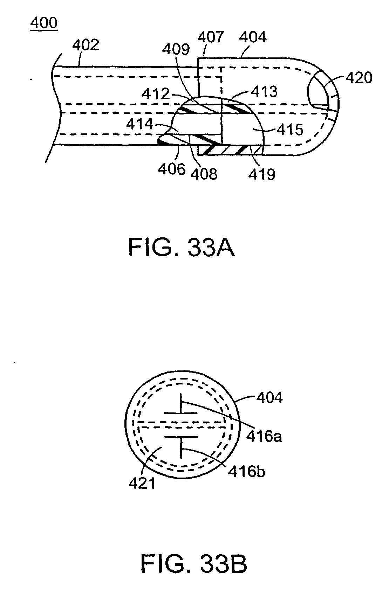

FIGS. 33A and 33B illustrate a medical device 400 that includes a catheter 402 and a cap 404 with a compound slit valve 416. The catheter 402 has an external surface 406 and two internal surfaces 408, 409 and defines two lumens 412, 414. The cap 404 has an external surface 407 and two internal surfaces 419, 420 and also defines two lumens 413, 415. The slit valves 416 depicted are T-shaped compound slits disposed on a distal end 421 of the cap 404. Slit 416a extends between external surface 407 and internal surface 420 of the cap 404 and facilitates communication between an environment external to the catheter 402 and lumens 412, 413. Slit 416b extends between external surface 407 and internal surface 419 of the cap 404 and facilitates communication between an environment external to the catheter 402 and lumens 414, 415. The cap 404 may be coupled to the catheter 402 by any of the methods described herein.

FIGS. 34A and 34B depict side and end views of an alternative embodiment of the medical device of FIGS. 33A and 33B, but without the cap 404. The catheter 402 has an external surface 406 and two internal surfaces 408, 409 and defines two lumens 412, 414. The slit valves 416 depicted are T-shaped compound slits disposed on a distal end 410 of the catheter 402. Slit 416a extends between external surface 406 and internal surface 408 of the catheter 402 and facilitates communication between an environment external to the catheter 402 and lumen 412. Slit 416b extends between external surface 406 and internal surface 409 of the catheter 402 and facilitates communication between an environment external to the catheter 402 and lumens 414.

FIGS. 35A-C depict various catheter and cap assemblies. In FIG. 35A, the catheter 382 is coupled to the cap 384 by a conduit 391. The conduit 391 has an outside diameter the same or slightly larger than the catheter's inside diameter. The conduit 391 is force fit within the lumen 383 of the catheter 382 and maintained in position by friction and/or an adhesive. The outside diameter of the conduit 391 is also the same or slightly larger than an inside diameter of the cap 384. The cap 384 is force fit over the conduit 391 and maintained in position by friction and/or an adhesive. The conduit 391 collects the catheter 382 and cap 384 and facilitates communication between the lumens 383, 389. FIG. 35B depicts the cap 384 force fit over an open end of the catheter 382, as also shown in FIG. 31A. The cap 3 84 has an inside diameter the same or smaller than the outside diameter of the catheter 382, and is secured to the catheter 382 by friction and/or an adhesive. FIG. 35C depicts a catheter 382 and cap 384 secured end to end. The inside diameters and/or outside diameters of the catheter 382 and cap 384 are approximately the same. The catheter 382 and cap 384 are secured at their abutting ends 393 by bonding, either by melting or chemical/solvent bonding.

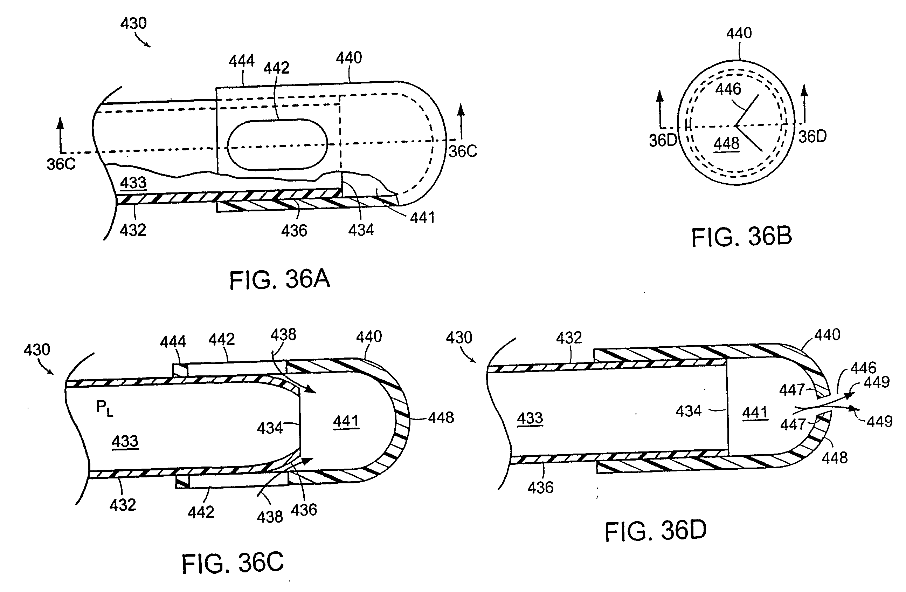

FIGS. 36A and 36B illustrate a medical device 430 that includes a catheter 432, a cap 440, and valves 442, 446. FIGS. 36C and 36D are cross-sectional views of FIGS. 36A and 36B, respectively, and illustrate the valves 442, 446 in operation. The catheter 432 is essentially the same as any of the catheters described herein, e.g., the catheter 432 defines a lumen 433. In addition, the catheter 432 defines an open distal end 434 and the wall 436 of the catheter 432 is designed to be collapsible at the open distal end 434 in response to a decrease in lumen pressure. The wall 436 of the catheter 432 can be designed to collapse by chemically weakening the wall 436, by manufacturing the catheter of variable durometers, or by manufacturing the catheter 432 with a reduced wall thickness in the area about the open distal end 434. In one embodiment, the wall 436 tapers down in the area around the open distal end 434 to improve the flexibility and collapsibility of the catheter 432 in the area of the distal open end 434. The cap 440 is essentially the same as the cap described with respect to FIGS. 31A and 31B; however, the cap 440 includes at least one slot valve 442 disposed on a proximal portion 444 of the cap 440. In the embodiment shown, the cap 440 defines a lumen 441 and two slots 444. The cap 440 is coupled to the catheter 432 in the manner described with respect to FIG. 35B. FIG. 36B is an end view of the catheter 432 and cap 440 assembly of FIG. 36A. The cap 440 has a nonlinear nonradial slit valve 446 disposed in the distal end 44S of the cap 440.

FIG. 36C depicts the operation of the catheter slit valve during aspiration. The pressure within the lumen (P_{L}) is decreased with respect to the pressure of the external environment (P_{E}), which results in the catheter wall 436 collapsing about the area of the open distal end 434. The collapse of the wall 436 causes the wall 434 to move away from the cap 440, thereby creating an opening between the cap 440 and the catheter wall 436 in the area of the slots 442. Fluid is then able to enter the lumens 433, 441 via the slots 442, as shown by arrows 438. Once the lumen pressure returns to normal, i.e., P_{L} is approximately equal to P_{E}, the catheter wall 436 returns to normal, thereby sealing with the cap 440 to close the slots 442.

FIG. 36D depicts the catheter slit valve during infusion. Infusion through the slit valve 446 is essentially the same as described hereinabove with respect to FIGS. 4A or 15B. Lumen pressure P_{L} is increased with respect to environmental pressure P_{E}, which causes the walls 447 adjacent the slit 446 to move apart, allowing infusion of fluid to the external environment, as shown by arrows 449.

The various catheters described herein may be manufactured by, for example, injection molding or modifying an extruded tube. For example, extrusion may be used to provide a uniform polymeric tube, to which a hub is attached at one end and the other end is sealed. Insert molding can then be used to provide the desired geometry of the slit regions. The slits could then be created in the desired valve locations as a subsequent mechanical operation. Insert molding allows the tip to be formed of a material either identical to or dissimilar from the catheter tube. The molded details in the protuberances include axial cross-sectional geometry, protuberance longitudinal cross-sectional geometry, protuberance length, wall thickness, degree of concave/convex curvature, etc. Other manufacturing techniques include melting or otherwise adhering the catheter portions, for example protuberances, as components or post-forming an extruded tube.

The biocompatible materials that can be used for the catheter include polymers, such as polymethanes, silicones, polyethylenes, nylons, polyesters, and polyester elastomers. The material hardness typically is between 40 and 100 durometer. The size and shape of the device may vary as necessary for a particular medical application. In one example, the overall OD of the catheter is about 3-16 French. The overall length of the catheter is about 20,3-102 cm (8-40 inches). For example, a 5 French catheter may have a substantially constant catheter body wall thickness, T (FIG. 22), of about 0,038 cm (0.015 inch) and a lumen diameter of about 0,089 cm about (0.035 inch). Protuberances are hemispherical, have a radial projection, P (FIG. 22), of about 0,038 cm (0.015 inch), a longitudinal extension, E (FIG. 28), of about 0.51 cm (0.200 inch) and a width, W (FIG. 22), between boundary inflection points of about 0.076 cm (0.030 inch). The slit may be formed by a cutting device, such as a custom shaped razor in conjunction with a holding fixture, and may have a length of 0.51 cm (0.200 inch). These dimensions are given for illustrative purposes only and are not meant to be limiting.

In additional embodiments, the catheter and valve types can be used in various combinations to create various combinations of infusion and aspiration effects using the principles illustrated above. A protuberance could be used on a catheter with a single slit valve. The protuberance may be trimmed manually by a physician prior to placement in the body to select the pressure differential needed to operate the valve. The protuberance could continue a distance along the catheter, for example, substantially the length of the catheter. In catheters with multiple valves, an infusion valve may be positioned proximal of an aspiration valve for, for example flushing the downstream area of the catheter including the aspiration valve area with a urokinase, to dissolve fibrin deposits. Alternatively, an infusion valve may be positioned distal of the aspiration valve, preferably near the distal end of the catheter, to facilitate flushing the full length of the lumen and avoiding dead volume. The catheters can be used in the vascular system for central venous access to deliver, for example, drugs to a cancer patient. The catheter can be placed by the Seldinger technique.

Having described certain embodiments of the invention, it will be apparent to those of ordinary skill in the art that other embodiments incorporating the concepts disclosed herein may be used without departing from the scope of the invention. The described embodiments are to be considered in all respects as only illustrative and not restrictive.

## Claims

1. A medical device (50) comprising:
an elongate catheter (52) including an external surface (56) and at least one internal surface (58) defining an internal lumen (60) that extends longitudinally along at least a portion of the elongate catheter (52); and
a nonradial slit (54, 74, 94, 114, 134, 154, 174, 200, 204, 222, 234, 254, 274, 294, 314, 336, 338, 386) **characterized by** the nonradial slit extending between end points separated along a portion of a length of the catheter (52), the nonradial slit extending through a wall of the catheter from the external surface (56) to the at least one internal surface (58) and into communication with the internal lumen (60), the nonradial slit being oriented so that, if a surface defined by the slit were extended through the catheter, the surface would not intersect an axis of the catheter.

2. A medical device according to claim 1, further comprising:
a protuberance (253) disposed on the elongate catheter (252) wherein the nonradial slit (54, 74, 94, 114, 134, 154, 174, 200, 204, 222, 234, 254, 274, 294, 314, 336, 338, 386) extends through the protuberance (253).

3. A medical device (290) according to claim 1 further comprising:
a protuberance (293) disposed on the elongate catheter (292), wherein the nonradial slit (54, 74, 94, 114, 134, 154, 174, 200, 204, 222, 234, 254, 274, 294, 314, 336, 338, 386) is disposed adjacent the protuberance (293).

4. A medical device (50) according to claim 1, wherein the slit (54, 74, 94, 114, 134, 154, 174, 200, 204, 222, 234, 254, 274, 294, 314, 336, 338, 386) forms an included angle (α) with a radial line (62) between about
(a) 1 degree and about 179 degrees;
(b) 10 degrees and about 40 degrees; and
(c) 30 degrees.

5. A medical device (50) according to claim 1,wherein the catheter (52) has a generally circular, oval, or rectangular cross-section.

6. A medical device (50) according to claim 1, wherein the slit (54, 74, 94, 114, 134, 154, 174, 200, 204, 222, 234, 254, 274, 294, 314, 336, 338, 386) is generally longitudinally disposed.

7. A medical device (50) according to claim 1, wherein the slit (54, 74, 94, 114, 134, 154, 174, 200, 204, 222, 234, 254, 274, 294, 314, 336, 338, 386) is between about
(a) 0.15 and about 2.5 centimeters in length (0.06 and about 1.0 inches in length);
(b) 0.3 and about 1.9 centimeters in length (0.12 and about 0.75 inches in length); and
(c) 0.38 and about 1 centimeter in length (0.15 and about 0.40 inches in length).

8. A medical device (220) according to claim 1, wherein the internal lumen (228) is eccentric with respect to the external surface (224).

9. A medical device (220) according to claim 8, wherein the nonradial slit (54, 74, 94, 114, 134, 154, 174, 200, 204, 222, 234, 254, 274, 294, 314, 336, 338, 386) is disposed in a thickened wall portion (223) of the catheter (221).

10. A medical device (50) according to claim 1, wherein the slit (54, 74, 94, 114, 134, 154, 174, 200, 204, 222, 234, 254, 274, 294, 314, 336, 338, 386) is linear.

11. A medical device (110) according to claim 1, wherein the slit (54, 74, 94, 114, 134, 154, 174, 200, 204, 222, 234, 254, 274, 294, 314, 336, 338, 386) is nonlinear, and comprises at least one angle (β) or a curve.

12. A medical device (130) according to claim 11, wherein the slit (54, 74, 94, 114, 134, 154, 174, 200, 204, 222, 234, 254, 274, 294, 314, 336, 338, 386) has a radius (R) of about 0.25 to about 1.27 centimeters (about 0.10 to about 0.50 inches).

13. A medical device (70) according to claim 1, further comprising a second nonradial slit (54, 74, 94, 114, 134, 154, 174, 200, 204, 222, 234, 254, 274, 294, 314, 336, 338, 386) extending from the external surface (76) to the at least one internal surface (78) and into communication with the internal lumen (80).

14. A medical device (70) according to claim 13, wherein the first and second slits (54, 74, 94, 114, 134, 154, 174, 200, 204, 222, 234, 254, 274, 294, 314, 336, 338, 386) are symmetrical about a radial line (82) and are arranged so as to converge at a point external to the elongate catheter (72).

15. A medical device (90) according to claim 13, wherein the first and second slits (54, 74, 94, 114, 134, 154, 174, 200, 204, 222, 234, 254, 274, 294, 314, 336, 338, 386) are symmetrical about a radial line (102) and are arranged so as to diverge from a point external to the elongate catheter (92).

16. A medical device (230) according to claim 5, further comprising a laminate (235) disposed on the external surface (236) and extending from the slit (54, 74, 94, 114, 134, 154, 174, 200, 204, 222, 234, 254, 274, 294, 314, 336, 338, 386) up to about
(a) 225 degrees from the slit (54, 74, 94, 114, 134, 154, 174, 200, 204, 222, 234, 254, 274, 294, 314, 336, 338, 386); and
(b) a laminate (235) disposed on the external surface (236) and extending from about 45 degrees from the slit (54, 74, 94, 114, 134, 154, 174, 200, 204, 222, 234, 254, 274, 294, 314, 336, 338, 386) up to about 225 degrees from the slit (54, 74, 94, 114, 134, 154, 174, 200, 204, 222, 234, 254, 274, 294, 314, 336, 338, 386).

17. A medical device (210) according to claim 13, wherein the elongate catheter (202) defines a second lumen (213).

18. A medical device (250) according to claim 2 or claim 3 wherein the protuberance (253) is disposed on the external surface (256) of the elongate catheter (252).

19. A medical device (270) according to claim 2 or claim 3, wherein the protuberance (273) is disposed on the at least one internal surface (278) of the elongate catheter (272).

20. A medical device (250) according to claim 2, further comprising:
a second protuberance (342) disposed on the elongate catheter (328); and
a second nonradial slit (54, 74, 94, 114, 134, 154, 174, 200, 204, 222, 234, 254, 274, 294, 314, 336, 338, 386) extending from the external surface (335), through the second protuberance (342) to the at least one internal surface (337), and into communication with the internal lumen (330).

21. A medical device (250) according to claim 20, wherein the first and second slits (54, 74, 94, 114, 134, 154, 174, 200, 204, 222, 234, 254, 274, 294, 314, 336, 338, 386) are symmetrical about a radial line and are arranged so as to converge at a point external to the elongate catheter (328).

22. A medical device (250) according to claim 20, wherein the first and second slits (54, 74, 94, 114, 134, 154, 174, 200, 204, 222, 234, 254, 274, 294, 314, 336, 338, 386) are symmetrical about a radial line and are arranged so as to diverge from a point external to the elongate catheter (328).

23. A medical device (250) according to claim 20, wherein the first and second protuberances (340, 342) are disposed opposite each other.

24. A medical device (250) according to claim 20, wherein the elongate catheter (328) defines a second lumen.

25. A medical device (368) according to claim 1, further comprising:
a further slit intersecting with the nonradial slit (54, 74, 94, 114, 134, 154, 174, 200, 204, 222, 234, 254, 274, 294, 314, 336, 338, 386) to form a compound slit (372).

26. A medical device (368) according to claim 25, wherein the compound slit (372) is disposed on a distal end of the elongate catheter (370).

27. A medical device (368) according to claim 26, further comprising a collar (375) disposed at the distal end of the catheter (370).

28. A medical device (368) according to claim 25, wherein the compound slit (372) is selected from:
(a) a tricuspid slit;
(b) a T-shaped slit:
(c) a cross-shaped slit; and
(d) a double T-shaped slit.

29. A medical device (400) according to claim 25, wherein the catheter (402) further comprises a second internal lumen (414) that extends longitudinally along at least a portion of the elongate catheter (402) and a second compound slit (416b) extending from the external surface (406) to the at least one internal surface (409) and into communication with the second internal lumen (414).

30. A medical device according to claim 1, further comprising a cap (384) including an external surface (387) and at least one internal surface (385) and defining a second lumen (389), the cap (384) being disposed at a distal end of the elongate catheter (382).

31. A medical device according to claim 2, further comprising a first protuberance (340) disposed on the elongate catheter (328);
the nonradial slit (54, 74, 94, 114, 134, 154, 174, 200, 204, 222, 234, 254, 274, 294, 314, 336, 338, 386) being disposed adjacent the first protuberance (340) and extending from the external surface (335) to one of the at least two internal surfaces (196), and into communication with the first internal lumen (198);
a second protuberance (342) disposed on the elongate catheter (328); and
a second slit (54, 74, 94, 114, 134, 154, 174, 200, 204, 222, 234, 254, 274, 294, 314, 336, 338, 386) disposed adjacent the second protuberance (342) and extending from the external surface (335) to another of the at least two internal surfaces (197), and into communication with the second internal lumen (199).

32. A medical device (290) according to claim 31, wherein the second slit (54, 74, 94, 114, 134, 154, 174, 200, 204, 222, 234, 254, 274, 294, 314, 336, 338, 386) is nonradial.

33. The medical device (430) of claim 31, wherein an open distal end (434) of the catheter (432) is collapsible.

34. The medical device (430) of claim 31, wherein the catheter (432) has a wall thickness that tapers down at an open distal end (434) of the catheter (432).

35. A medical device (50) according to claim 1, wherein the surface forms a part of a plane which is parallel to the axis of the catheter and which does not intersect the axis of the catheter.

## Patentansprüche

1. Medizinische Vorrichtung (50), aufweisend:
einen länglichen Katheder (52) mit einer Außenseite (56) und wenigstens einer Innenseite (58), die ein inneres Lumen (60) definiert, das sich in Längsrichtung entlang wenigstens eines Teils des länglichen Katheders (52) erstreckt; und
einen nicht-radialen Schlitz (54, 74, 94,114,134,154,174,200,204, 222,234, 254, 274, 294, 314, 336, 338, 386),
**dadurch kennzeichnet, dass**
der nicht-radiale Schlitz sich zwischen Endpunkten erstreckt, die entlang eines Teils einer Länge des Katheder (52) getrennt sind, wobei der nicht-radiale Schlitz durch eine Wand des Katheders von der Außenseite (56) zu der wenigstens einen Innenseite (58) verläuft und in Verbindung mit dem inneren Lumen (60) ist, wobei der nicht-radiale Schlitz so ausgerichtet ist, dass, wenn eine durch den Schlitz definierte Fläche durch den Katheder verlängert werden würde, die Fläche eine Achse des Katheders nicht schneidet.

2. Medizinische Vorrichtung gemäß Anspruch 1, weiterhin aufweisend:
eine Ausstülpung (253), die an dem länglichen Katheder (252) angeordnet ist, wobei der nicht-radiale Schlitz (54, 74, 94, 114, 134, 154, 174,200, 204, 222, 234, 254, 274, 294, 314, 336, 338, 386) sich durch die Ausstülpung (253) erstreckt.

3. Medizinische Vorrichtung (290) gemäß Anspruch 1, weiterhin aufweisend:
eine Ausstülpung (293), die an dem länglichen Katheder (292) angeordnet ist, wobei der nicht-radiale Schlitz (54, 74, 94, 114,134, 154, 174, 200, 204, 222, 234, 254, 274, 294, 314, 336, 338, 386) angrenzend an die Ausstülpung (293) angeordnet ist.

4. Medizinische Vorrichtung (50) gemäß Anspruch 1, wobei der Schlitz (54, 74, 94, 114, 134, 154, 174, 200, 204, 222, 234, 254, 274, 294, 314, 336, 338, 386) mit einer radialen Linie (62) einen eingeschlossenen Winkel (α) bildet, zwischen etwa
(a) 1 Grad und etwa 179 Grad;
(b) 10 Grad und etwa 40 Grad; und
(c) 30 Grad.

5. Medizinische Vorrichtung (50) gemäß Anspruch 1, wobei der Katheder (52) einen allgemein kreisförmigen, ovalen oder rechtwinkligen Querschnitt aufweist.

6. Medizinische Vorrichtung (50) gemäß Anspruch 1, wobei der Schlitz (54, 74, 94, 114, 134, 154, 174, 200, 204, 222, 234, 254, 274, 294, 314, 336, 338, 386) allgemein in Längsrichtung angeordnet ist.

7. Medizinische Vorrichtung (50) gemäß Anspruch 1, wobei der Schlitz (54, 74, 94, 114, 134, 154, 174, 200, 204, 222, 234, 254, 274, 294, 314, 336, 338, 386) zwischen etwa
(a) 0,15 und 2,5 cm lang (0,06 und etwa 1,0 Zoll lang);
(b) 0,3 und etwa 1,9 cm lang (0,12 und etwa 0,75 Zoll lang); und (c) 0,38 cm und etwa 1 cm lang (0,15 und etwa 0,40 Zoll lang) ist.

8. Medizinische Vorrichtung (220) gemäß Anspruch 1, wobei das innere Lumen (228) exzentrisch bezüglich der Außenseite (224) ist.

9. Medizinische Vorrichtung (220) gemäß Anspruch 8, wobei der nicht-radiale Schlitz (54, 74, 94, 114, 134, 154, 174, 200, 204, 222, 234, 254, 274, 294, 314, 336, 338, 386) in einem verdickten Wandabschnitt (223) des Katheders (221) angeordnet ist.

10. Medizinische Vorrichtung (50) gemäß Anspruch 1, wobei der Schlitz (54, 74, 94, 114, 134, 154, 174, 200, 204, 222, 234, 254, 274, 294, 314, 336, 338, 386) linear ist.

11. Medizinische Vorrichtung (110) gemäß Anspruch 1, wobei der Schlitz (54, 74, 94, 114, 134, 154, 174, 200, 204, 222, 234, 254, 274, 294, 314, 336, 338, 386) nicht-linear ist und mindestens einen Winkel (β) oder eine Krümmung aufweist.

12. Medizinische Vorrichtung (130) gemäß Anspruch 11, wobei der Schlitz (54, 74, 94, 114, 134, 154, 174, 200, 204, 222, 234, 254, 274, 294, 314, 336, 338, 386) einen Radius (R) von etwa 0,25 bis etwa 1,27 cm (etwa 0,10 bis etwa 0,50 Zoll) besitzt.

13. Medizinische Vorrichtung (70) gemäß Anspruch 1, weiterhin einen zweiten nicht-radialen Schlitz (54, 74, 94, 114, 134, 154, 174, 200, 204, 222, 234, 254, 274, 294,314,336, 338, 386) aufweisend, der sich von der Außenseite (76) zu der wenigstens einen Innenseite (78) erstreckt und mit dem inneren Lumen (80) in Verbindung ist.

14. Medizinische Vorrichtung (70) gemäß Anspruch 13, wobei der erste und der zweite Schlitz (54, 74, 94, 114, 134, 154, 174, 200, 204, 222, 234, 254, 274, 294, 314, 336, 338, 386) um eine radiale Linie (82) symmetrisch sind und so angeordnet sind, dass sie an einem Punkt außerhalb des länglichen Katheders (72) konvergieren.

15. Medizinische Vorrichtung (90) gemäß Anspruch 13, wobei der erste und der zweite Schlitz (54, 74, 94, 114, 134, 154, 174, 200, 204, 222, 234, 254, 274, 294, 314, 336, 338, 386) um eine radiale Linie (102) symmetrisch sind und so angeordnet sind, dass sie von einem Punkt außerhalb des länglichen Katheders (92) divergieren.

16. Medizinische Vorrichtung (230) gemäß Anspruch 5, weiterhin aufweisend einen Schichtstoff (23 5), der auf der Außenseite (236) angeordnet ist und sich von dem Schlitz (54, 74, 94, 114, 134, 154, 174, 200, 204, 222, 234, 254, 274, 294, 314, 336, 338, 386) bis zu etwa
(a) 225 Grad von dem Schlitz (54, 74, 94, 114, 134, 154, 174, 200, 204, 222, 234, 254, 274, 294, 314, 336, 338, 386) erstreckt; und
(b) ein Schichtstoff (235), der auf der Außenseite (236) angeordnet ist und sich von etwa 45 Grad von dem Schlitz (54, 74, 94, 114, 134,154,174, 200, 204, 222, 234, 254, 274, 294, 314, 336, 338, 386) bis zu etwa 225 Grad von dem Schlitz (54, 74, 94, 114, 134, 154, 174, 200, 204, 222, 234, 254, 274, 294, 314, 336, 338, 386) erstreckt.

17. Medizinische Vorrichtung (210) gemäß Anspruch 13, wobei der längliche Katheder (202) ein zweites Lumen (213) definiert.

18. Medizinische Vorrichtung (250) gemäß Anspruch 2 oder 3, wobei die Ausstülpung (253) an der Außenseite (256) des länglichen Katheder (252) angeordnet ist.

19. Medizinische Vorrichtung (270) gemäß Anspruch 2 oder 3, wobei die Ausstülpung (273) an wenigstens einer Innenseite (278) des länglichen Katheders (272) angeordnet ist

20. Medizinische Vorrichtung (250) gemäß Anspruch 2, weiterhin aufweisend:
eine zweiten Ausstülpung (342), die am länglichen Katheder (328) angeordnet ist; und
einen zweiten nicht-radialen Schlitz (54, 74, 94, 114, 134, 154, 174, 200, 204, 222, 234, 254, 274, 294, 314, 336, 338, 386), der sich von der Außenseite (335) durch die zweite Ausstülpung (342) zu der wenigstens einen Innenseite (337) erstreckt und mit dem inneren Lumen (330) verbunden ist.

21. Medizinische Vorrichtung (250) gemäß Anspruch 20, wobei der erste und der zweite Schlitz (54, 74, 94, 114, 134, 154, 174, 200, 204, 222, 234, 254, 274, 294, 314, 336, 338, 386) um eine radiale Linie symmetrisch sind und so angeordnet sind, dass sie an einem Punkt außerhalb des länglichen Katheders (328) konvergieren.

22. Medizinische Vorrichtung (250) gemäß Anspruch 20, wobei der erste und der zweite Schlitz (54, 74, 94, 114, 134, 154, 174, 200, 204, 222, 234, 254, 274, 294, 314, 336, 338, 386) um eine radiale Linie symmetrisch sind und so angeordnet sind, dass sie von einem Punkt außerhalb des länglichen Katheders (328) divergieren.

23. Medizinische Vorrichtung (250) gemäß Anspruch 20, wobei die erste und zweite Ausstülpung (340, 342) zueinander abgewandt angeordnet sind.

24. Medizinische Vorrichtung (250) gemäß Anspruch 20, wobei der Längliche Katheder (328) ein zweites Lumen definiert.

25. Medizinische Vorrichtung (368) gemäß Anspruch 1, weiterhin aufweisend: einen weiteren Schlitz, der sich mit dem nicht-radialen Schlitz (54, 74, 94, 114, 134, 154, 174, 200, 204, 222, 234, 254, 274, 294, 314, 336, 338, 386) schneidet, um einen zusammengesetzten Schlitz (372) zu bilden.

26. Medizinische Vorrichtung (368) gemäß Anspruch 25, wobei der zusammengesetzte Schlitz (372) an einem distalen Ende des länglichen Katheders (370) angeordnet ist.

27. Medizinische Vorrichtung (368) gemäß Anspruch 26, weiterhin einen Kragen (375) aufweisend, der am distalen Ende des Katheders (370) angeordnet ist.

28. Medizinische Vorrichtung (368) gemäß Anspruch 25, wobei der zusammengesetzte Schlitz (372) ausgewählt ist aus:
(a) einem dreispitzigen Schlitz;
(b) einem T-förmigen Schlitz:
(c) einem kreuzförmigen Schlitz; und
(d) einem Doppel-T-förmigen Schlitz.

29. Medizinische Vorrichtung (400) gemäß Anspruch 25, wobei der Katheder (402) weiterhin ein zweites inneres Lumen (414) aufweist, das sich in Längsrichtung wenigstens entlang einem Teil des länglichen Katheders (402) erstreckt, und einen zweiten zusammengesetzten Schlitz (416b), der sich von der Außenseite (406) zu der wenigstens einen Innenseite (409) erstreckt und in Verbindung mit dem zweiten inneren Lumen (414) steht.

30. Medizinische Vorrichtung gemäß Anspruch 1, weiterhin aufweisend eine Kappe (384) mit einer Außenseite (387) und wenigstens einer Innenseite (385) und ein zweites Lumen (389) definierend, wobei die Kappe (384) an einem distalen Ende des länglichen Katheders (382) angeordnet ist.

31. Medizinische Vorrichtung gemäß Anspruch 2, weiterhin aufweisend eine erste Ausstülpung (340), die am länglichen Katheder (328) angeordnet ist;
wobei der nicht-radiale Schlitz (54, 74, 94, 114, 134, 154, 174, 200, 204, 222, 234, 254, 274, 294, 314, 336, 338, 386) angrenzend an die erste Ausstülpung (340) angeordnet ist und sich von der Außenseite (335) zu einer der wenigstens zwei Innenseiten (196) erstreckt und mit dem ersten inneren Lumen (330) in Verbindung ist.
eine zweite Ausstülpung (342) aufweisend, die am länglichen Katheder (328) angeordnet ist; und einen zweiten Schlitz (54, 74, 94, 114, 134, 154, 174, 200, 204, 222, 234, 254, 274, 294, 314, 336, 338, 386), der angrenzend an die zweite Ausstülpung (342) angeordnet ist und sich von der Außenseite (335) zur anderen der wenigstens zwei Innenseiten (197) erstreckt und mit dem zweiten inneren Lumen (199) verbunden ist.

32. Medizinische Vorrichtung (290) gemäß Anspruch 31, wobei der zweite Schlitz (54, 74, 94, 114, 134, 154, 174, 200, 204, 222, 234, 254, 274, 294, 314, 336, 338, 386) nicht-radial ist.

33. Medizinische Vorrichtung (430) gemäß Anspruch 31, wobei ein offenes distales Ende (434) des Katheters (432) zusammenklappbar ist.

34. Medizinische Vorrichtung (430) gemäß Anspruch 31, wobei der Katheder (432) eine Wanddicke aufweist, die sich hin zu einem offenen distalen Ende (434) des Katheters (432) verjüngt.

35. Medizinische Vorrichtung (50) gemäß Anspruch 1, wobei die Fläche einen Teil einer Ebene bildet, die parallel zur Kathederachse ist und die die Kathederachse nicht schneidet.

## Revendications

1. Dispositif médical (50) comprenant :
un cathéter allongé (52) comprenant une surface externe (56) et au moins une surface interne (58) définissant une lumière interne (60) qui s'étend longitudinalement le long d'au moins une partie du cathéter allongé (52) ; et
une fente non radiale (54, 74, 94, 114, 134, 154, 174, 200, 204, 222, 234, 254, 274, 294, 314, 336, 338, 386), **caractérisé en ce que** la fente non radiale s'étend entre des points terminaux espacés le long d'une partie d'une longueur du cathéter (52), la fente non radiale s'étendant au travers d'une paroi du cathéter de la surface externe (56) jusqu'à l'au moins une surface interne (58) et en communication avec la lumière interne (60), la fente non radiale étant orientée de telle sorte que si une surface définie par la fente s'étendait au travers du cathéter, la surface ne croiserait pas un axe du cathéter.

2. Dispositif médical selon la revendication 1, comprenant en outre :
une protubérance (253) disposée sur le cathéter allongé (252), la fente non radiale (54, 74, 94, 114, 134, 154, 174, 200, 204, 222, 234, 254, 274, 294, 314, 336, 338, 386) s'étendant au travers de la protubérance (253).

3. Dispositif médical (290) selon la revendication 1, comprenant en outre :
une protubérance (293) disposée sur le cathéter allongé (292), la fente non radiale (54, 74, 94, 114, 134, 154, 174, 200, 204, 222, 234, 254, 274, 294, 314, 336, 338, 386) étant disposée au voisinage immédiat de la protubérance (293).

4. Dispositif médical (50) selon la revendication 1, dans lequel la fente (54, 74, 94, 114, 134, 154, 174, 200, 204, 222, 234, 254, 274, 294, 314, 336, 338, 386) forme un angle au sommet (α) avec une ligne radiale (62) compris entre environ
(a) 1 degré et environ 179 degrés
(b) 10 degrés et environ 40 degrés ; et (c) 30 degrés.

5. Dispositif médical (50) selon la revendication 1, dans lequel le cathéter (52) a une section globalement circulaire, ovale ou rectangulaire.

6. Dispositif médical (50) selon la revendication 1, dans lequel la fente (54, 74, 94, 114, 134, 154, 174, 200, 204, 222, 234, 254, 274, 294, 314, 336, 338, 386) est globalement disposée longitudinalement.

7. Dispositif médical (50) selon la revendication 1, dans lequel la fente (54, 74, 94, 114, 134, 154, 174, 200, 204, 222, 234, 254, 274, 294, 314, 336, 338, 386) mesure environ entre
(a) 0,15 et environ 2,5 centimètres de longueur (0,06 et environ 1,0 pouces de longueur) ;
(b) 0,3 et environ 1,9 centimètres de longueur (0,12 et environ 0,75 pouces de longueur) ; et
(c) 0,38 et environ 1 centimètre de longueur (0,15 et environ 0,40 pouces de longueur.

8. Dispositif médical (220) selon la revendication 1, dans lequel la lumière interne (228) est excentrique par rapport à la surface externe (224).

9. Dispositif médical (220) selon la revendication 8, dans lequel la fente non radiale (54, 74, 94, 114, 134, 154, 174, 200, 204, 222, 234, 254, 274, 294, 314, 336, 338, 386) est disposée dans une partie de paroi élargie (223) du cathéter (221).

10. Dispositif médical (50) selon la revendication 1, dans lequel la fente (54, 74, 94, 114, 134, 154, 174, 200, 204, 222, 234, 254, 274, 294, 314, 336, 338, 386) est linéaire.

11. Dispositif médical (110) selon la revendication 1, dans lequel la fente (54, 74, 94, 114, 134, 154, 174, 200, 204, 222, 234, 254, 274, 294, 314, 336, 338, 386) est non linéaire et comprend au moins un angle (β) ou une courbe.

12. Dispositif médical (130) selon la revendication 11, dans lequel la fente (54, 74, 94, 114, 134, 154, 174, 200, 204, 222, 234, 254, 274, 294, 314, 336, 338, 386) a un rayon (R) d'environ 0,25 à environ 1,27 centimètres (environ 0,10 à environ 0,50 pouces).

13. Dispositif médical (70) selon la revendication 1, comprenant en outre une deuxième fente non radiale (54, 74, 94, 114, 134, 154, 174, 200, 204, 222, 234, 254, 274, 294, 314, 336, 338, 386) s'étendant de la surface externe (76) jusqu'à l'au moins une surface interne (78) et en communication avec la lumière interne (80).

14. Dispositif médical (70) selon la revendication 13, dans lequel les première et deuxième fentes (54, 74, 94, 114, 134, 154, 174, 200, 204, 222, 234, 254, 274, 294, 314, 336, 338, 386) sont symétriques autour d'une ligne radiale (82) et sont disposées de façon à converger en un point extérieur au cathéter allongé (72).

15. Dispositif médical (90) selon la revendication 13, dans lequel les première et deuxième fentes (54, 74, 94, 114, 134, 154, 174, 200, 204, 222, 234, 254, 274, 294, 314, 336, 338, 386) sont symétriques autour d'une ligne radiale (102) et sont disposées de façon à diverger depuis un point extérieur au cathéter allongé (92).

16. Dispositif médical (230) selon la revendication 5, comprenant en outre un stratifié (235) disposé sur la surface externe (236) et s'étendant depuis la fente (54, 74, 94, 114, 134, 154, 174, 200, 204, 222, 234, 254, 274, 294, 314, 336, 338, 386) jusqu'à environ
(a) 225 degrés de la fente (54, 74, 94, 114, 134, 154, 174, 200, 204, 222, 234, 254, 274, 294, 314, 336, 338, 386) ; et
(b) un stratifié (235) disposé sur la surface externe (236) et s'étendant d'environ 45 degrés de la fente (54, 74, 94, 114, 134, 154, 174, 200, 204, 222, 234, 254, 274, 294, 314, 336, 338, 386) jusqu'à environ 225 degrés de la fente (54, 74, 94, 114, 134, 154, 174, 200, 204, 222, 234, 254, 274, 294, 314, 336, 338, 386).

17. Dispositif médical (210) selon la revendication 13, dans lequel le cathéter allongé (202) définit une seconde lumière (213).

18. Dispositif médical (250) selon la revendication 2 ou 3, dans lequel la protubérance (253) est disposée sur la surface externe (256) du cathéter allongé (252).

19. Dispositif médical (270) selon la revendication 2 ou 3, dans lequel la protubérance (273) est disposée sur l'au moins une surface interne (278) du cathéter allongé (272).

20. Dispositif médical (250) selon la revendication 2, comprenant en outre :
une seconde protubérance (342) disposée sur le cathéter allongé (328) ; et
une deuxième fente non radiale (54, 74, 94, 114, 134, 154, 174, 200, 204, 222, 234, 254, 274, 294, 314, 336, 338, 386) s'étendant depuis la surface externe (335), en passant par la seconde protubérance (342) jusqu'à l'au moins une surface interne (337) et en communication avec la lumière interne (330).

21. Dispositif médical (250) selon la revendication 20, dans lequel les première et deuxième fentes (54, 74, 94, 114, 134, 154, 174, 200, 204, 222, 234, 254, 274, 294, 314, 336, 338, 386) sont symétriques autour d'une ligne radiale et sont disposées de façon à converger en un point extérieur au cathéter allongé (328).

22. Dispositif médical (250) selon la revendication 20, dans lequel les première et deuxième fentes (54, 74, 94, 114, 134, 154, 174, 200, 204, 222, 234, 254, 274, 294, 314, 336, 338, 386) sont symétriques autour d'une ligne radiale et sont disposées de façon à diverger depuis un point extérieur au cathéter allongé (328).

23. Dispositif médical (250) selon la revendication 20, dans lequel les première et seconde protubérances (340, 342) sont disposées de façon opposée l'une de l'autre.

24. Dispositif médical (250) selon la revendication 20, dans lequel le cathéter allongé (328) définit une seconde lumière.

25. Dispositif médical (368) selon la revendication 1, comprenant en outre :
une autre fente croisant la fente non radiale (54, 74, 94, 114, 134, 154, 174, 200, 204, 222, 234, 254, 274, 294, 314, 336, 338, 386) pour former une fente composée (372).

26. Dispositif médical (368) selon la revendication 25, dans lequel la fente composée (372) est disposée sur une extrémité distale du cathéter allongé (370).

27. Dispositif médical (368) selon la revendication 26, comprenant en outre un collier (375) disposé au niveau de l'extrémité distale du cathéter (370).

28. Dispositif médical (368) selon la revendication 25, dans lequel la fente composée (372) est choisie parmi :
(a) une fente tricuspide ;
(b) une fente en T ;
(c) une fente en croix ; et
(d) une fente en double T.

29. Dispositif médical (400) selon la revendication 25, dans lequel le cathéter (402) comprend en outre une seconde lumière interne (414) qui s'étend longitudinalement le long d'au moins une partie du cathéter allongé (402) et une deuxième fente composée (416b) s'étendant depuis la surface externe (406) à l'au moins une surface interne (409) et en communication avec la seconds lumière interne (414).

30. Dispositif médical selon la revendication 1, comprenant en outre un couvercle (384) comprenant une surface externe (387) et au moins une surface interne (385) et définissant une seconde lumière (389), le couvercle (384) étant disposé à une extrémité distale du cathéter allongé (382).

31. Dispositif médical selon la revendication 2, comprenant en outre une première protubérance (340) disposée sur le cathéter allongé (328) ;
la fente non radiale (54, 74, 94, 114, 134, 154, 174, 200, 204, 222, 234, 254, 274, 294, 314, 336, 338, 386) étant disposée au voisinage immédiat de la première protubérance (340) et s'étendant depuis la surface externe (335) à l'une des au moins deux surfaces internes (196) et en communication avec la première lumière interne (198) ;
une seconde protubérance (342) disposée sur le cathéter allongé (328) ; et
une deuxième fente (54, 74, 94, 114, 134, 154, 174, 200, 204, 222, 234, 254, 274, 294, 314, 336, 338, 386) disposée au voisinage immédiat de la seconde protubérance (342) et s'étendant depuis la surface externe (335) à une autre des au moins deux surfaces internes (197) et en communication avec la seconde lumière interne (199).

32. Dispositif médical (290) selon la revendication 31, dans lequel la deuxième fente (54, 74, 94, 114, 134, 154, 174, 200, 204, 222, 234, 254, 274, 294, 314, 336, 338, 386) est non radiale.

33. Dispositif médical (430) selon la revendication 31, dans lequel une extrémité distale ouverte (434) du cathéter (432) peut être repliée.

34. Dispositif médical (430) selon la revendication 31, dans lequel le cathéter (432) a une épaisseur de paroi qui s'affine au niveau d'une extrémité distale ouverte (434) du cathéter (432).

35. Dispositif médical (50) selon la revendication 1, dans lequel la surface forme une partie d'un plan qui est parallèle à l'axe du cathéter et qui ne croise pas l'axe du cathéter.
